# EUROPEAN PATENT SPECIFICATION

(11) **EP 3 095 869 B1**
(45) Date of publication and mention of the grant of the patent: **19.06.2019**
(21) Application number: 16167953.5
(22) Date of filing: 10.04.2009
(51) Int. Cl.: C12N 15/53, C12N 9/02, C12N 15/62, C12N 15/82, C12N 5/14

(54) **MUTATED HYDROXYPHENYLPYRUVATE DIOXYGENASE, DNA SEQUENCE AND ISOLATION OF PLANTS WHICH ARE TOLERANT TO HPPD INHIBITOR HERBICIDES**
MUTIERTE HYDROXYPHENYLPYRUVATDIOXYGENASE, DNA-SEQUENZ UND ISOLIERUNG VON GEGEN HPPD-HEMMER-HERBIZIDE RESISTENTEN PFLANZEN
NOUVELLE DIOXYGÉNASE D'HYDROXYPHÉNYLPYRUVATE MUTÉE, SÉQUENCE D'ADN ET ISOLATION DE PLANTES QUI SONT TOLÉRANTES À DES HERBICIDES INHIBANT L'HPPD

(30) Priority: 14.04.2008 EP 08154481; 14.04.2008 US 124082 P
(43) Date of publication of application: 23.11.2016
(62) Divisional of application: 09753734.4
(73) Proprietor: BASF Agricultural Solutions Seed US LLC, Florham Park, NJ 07932 (US)
(72) Inventor: BUSCH, Marco, 65527 Niedernhausen-Oberjosbach (DE); FISCHER, Kerstin, 55232 Alzey-Weinheim (DE); LABER, Bernd, 65510 Idstein (DE); SAILLAND, Alain, Raleigh NC, 27606 (US)
(74) Representative: J A Kemp

(56) References cited:
- WO-A-02/46387
- WO-A-96/38567
- WO-A-97/49816
- WO-A-98/04685
- WO-A-99/24585
- WO-A-2004/024928
- MATRINGE MICHEL ET AL: "p-hydroxyphenylpyruvate dioxygenase inhibitor-resistant plants", PEST MANAGEMENT SCIENCE, vol. 61, no. 3, March 2005 (2005-03), pages 269-276, XP002511974, ISSN: 1526-498X
- M.D.K. OWEN ET AL: "2008 HERBICIDE GUIDE FOR IOWA CORN AND SOYBEAN PRODUCTION", , November 2007 (2007-11), pages 1-15, XP002498361, Retrieved from the Internet: URL:http://www.weeds.iastate.edu/reference /08WC94.pdf [retrieved on 2008-10-02]
- FRITZE IRIS M ET AL: "The crystal structures of Zea mays and Arabidopsis 4-hydroxyphenylpyruvate dioxygenase", PLANT PHYSIOLOGY (ROCKVILLE), vol. 134, no. 4, April 2004 (2004-04), pages 1388-1400, XP002511973, ISSN: 0032-0889
- KAKIDANI HITOSHI ET AL: "Three-dimensional modeling of plant 4-hydroxyphenylpyruvate dioxygenase, a molecular target of triketone-type herbicides", JOURNAL OF PESTICIDE SCIENCE - NIPPON NOYAKU GAKKAISHI, NIPPON NOYAKU GAKKAI, TOKYO, JP, vol. 28, no. 4, 1 January 2003 (2003-01-01), pages 409-415, XP008100740, ISSN: 0385-1559

### SEQUENCE LISTING

<110> Bayer Cropscience AG Bayer Bioscience NV
<120> New mutated hydroxyphenylpyruvate dioxygenase, DNA sequence and isolation of plants which are tolerant to HPPD herbicides
<130> BCS 08-4008
<160> 32
<170> PatentIn version 3.4
<210> 1
   <211> 1077
   <212> DNA
   <213> Pseudomonas fluorescens
<220>
   <221> CDS
   <222> (1)..(1077)
<400> 1
   atg gca gat cta tac gaa aac cca atg ggc ctg atg ggc ttt gaa ttc      48
   Met Ala Asp Leu Tyr Glu Asn Pro Met Gly Leu Met Gly Phe Glu Phe
   1               5                   10                  15
   atc gaa ttc gcg tcg ccg acg ccg ggt acc ctg gag ccg atc ttc gag      96
   Ile Glu Phe Ala Ser Pro Thr Pro Gly Thr Leu Glu Pro Ile Phe Glu
               20                  25                  30
   atc atg ggc ttc acc aaa gtc gcg acc cac cgt tcc aag aac gtg cac     144
   Ile Met Gly Phe Thr Lys Val Ala Thr His Arg Ser Lys Asn Val His
           35                  40                  45
   ctg tac cgc cag ggc gag atc aac ctg atc ctc aac aac gag ccc aac     192
   Leu Tyr Arg Gln Gly Glu Ile Asn Leu Ile Leu Asn Asn Glu Pro Asn
       50                  55                  60
   agc atc gcc tcc tac ttt gcg gcc gaa cac ggc ccg tcg gtg tgc ggc     240
   Ser Ile Ala Ser Tyr Phe Ala Ala Glu His Gly Pro Ser Val Cys Gly
   65                  70                  75                  80
   atg gcg ttc cgc gtg aag gac tcg caa aag gcc tac aac cgc gcc ctg     288
   Met Ala Phe Arg Val Lys Asp Ser Gln Lys Ala Tyr Asn Arg Ala Leu
                   85                  90                  95
   gaa ctc ggc gcc cag ccg atc cat att gac acc ggg ccg atg gaa ttg     336
   Glu Leu Gly Ala Gln Pro Ile His Ile Asp Thr Gly Pro Met Glu Leu
               100                 105                 110
   aac ctg ccg gcg atc aag ggc atc ggc ggc gcg ccg ttg tac ctg atc     384
   Asn Leu Pro Ala Ile Lys Gly Ile Gly Gly Ala Pro Leu Tyr Leu Ile
           115                 120                 125
   gac cgt ttc ggc gaa ggc agc tcg atc tac gac atc gac ttc gtg tac     432
   Asp Arg Phe Gly Glu Gly Ser Ser Ile Tyr Asp Ile Asp Phe Val Tyr
       130                 135                 140
   ctc gaa ggt gtg gag cgc aat ccg gtc ggt gca ggt ctc aaa gtc atc     480
   Leu Glu Gly Val Glu Arg Asn Pro Val Gly Ala Gly Leu Lys Val Ile
   145                 150                 155                 160
   gac cac ctg acc cac aac gtc tat cgc ggc cgc atg gtc tac tgg gcc       528
   Asp His Leu Thr His Asn Val Tyr Arg Gly Arg Met Val Tyr Trp Ala
                   165                 170                 175
   aac ttc tac gag aaa ttg ttc aac ttc cgt gaa gcg cgt tac ttc gat       576
   Asn Phe Tyr Glu Lys Leu Phe Asn Phe Arg Glu Ala Arg Tyr Phe Asp
               180                 185                 190
   atc aag ggc gag tac acc ggc ctg act tcc aag gcc atg agt gcg ccg       624
   Ile Lys Gly Glu Tyr Thr Gly Leu Thr Ser Lys Ala Met Ser Ala Pro
           195                 200                 205
   gac ggc atg atc cgc atc ccg ctg aac gaa gag tcg tcc aag ggc gcg       672
   Asp Gly Met Ile Arg Ile Pro Leu Asn Glu Glu Ser Ser Lys Gly Ala
       210                 215                 220
   ggg cag atc gaa gag ttc ctg atg cag ttc aac ggc gaa ggc atc cag       720
   Gly Gln Ile Glu Glu Phe Leu Met Gln Phe Asn Gly Glu Gly Ile Gln
   225                 230                 235                 240
   cac gtg gcg ttc ctc acc gac gac ctg gtc aag acc tgg gac gcg ttg       768
   His Val Ala Phe Leu Thr Asp Asp Leu Val Lys Thr Trp Asp Ala Leu
                   245                 250                 255
   aag aaa atc ggc atg cgc ttc atg acc gcg ccg cca gac act tat tac       816
   Lys Lys Ile Gly Met Arg Phe Met Thr Ala Pro Pro Asp Thr Tyr Tyr
               260                 265                 270
   gaa atg ctc gaa ggc cgc ctg cct gac cac ggc gag ccg gtg gat caa       864
   Glu Met Leu Glu Gly Arg Leu Pro Asp His Gly Glu Pro Val Asp Gln
           275                 280                 285
   ctg cag gca cgc ggt atc ctg ctg gac gga tct tcc gtg gaa ggc gac       912
   Leu Gln Ala Arg Gly Ile Leu Leu Asp Gly Ser Ser Val Glu Gly Asp
       290                 295                 300
   aaa cgc ctg ctg ctg cag atc ttc tcg gaa acc ctg atg ggc ccg gtg       960
   Lys Arg Leu Leu Leu Gln Ile Phe Ser Glu Thr Leu Met Gly Pro Val
   305                 310                 315                 320
   ttc ttc gaa ttc atc cag cgc aag ggc gac gat ggg ttt ggc gag ggg      1008
   Phe Phe Glu Phe Ile Gln Arg Lys Gly Asp Asp Gly Phe Gly Glu Gly
                   325                 330                 335
   aac ttc aag gcg ctg ttc gag tcc atc gaa cgt gac cag gtg cgt cgt      1056
   Asn Phe Lys Ala Leu Phe Glu Ser Ile Glu Arg Asp Gln Val Arg Arg
               340                 345                 350
   ggt gta ttg acc gcc gat taa                                          1077
   Gly Val Leu Thr Ala Asp
           355
<210> 2
   <211> 358
   <212> PRT
   <213> Pseudomonas fluorescens
<400> 2
   Met Ala Asp Leu Tyr Glu Asn Pro Met Gly Leu Met Gly Phe Glu Phe
   1               5                   10                  15
   Ile Glu Phe Ala Ser Pro Thr Pro Gly Thr Leu Glu Pro Ile Phe Glu
               20                  25                  30
   Ile Met Gly Phe Thr Lys Val Ala Thr His Arg Ser Lys Asn Val His
           35                  40                  45
   Leu Tyr Arg Gln Gly Glu Ile Asn Leu Ile Leu Asn Asn Glu Pro Asn
       50                  55                  60
   Ser Ile Ala Ser Tyr Phe Ala Ala Glu His Gly Pro Ser Val Cys Gly
   65                  70                  75                  80
   Met Ala Phe Arg Val Lys Asp Ser Gln Lys Ala Tyr Asn Arg Ala Leu
                   85                  90                  95
   Glu Leu Gly Ala Gln Pro Ile His Ile Asp Thr Gly Pro Met Glu Leu
               100                 105                 110
   Asn Leu Pro Ala Ile Lys Gly Ile Gly Gly Ala Pro Leu Tyr Leu Ile
           115                 120                 125
   Asp Arg Phe Gly Glu Gly Ser Ser Ile Tyr Asp Ile Asp Phe Val Tyr
       130                 135                 140
   Leu Glu Gly Val Glu Arg Asn Pro Val Gly Ala Gly Leu Lys Val Ile
   145                 150                 155                 160
   Asp His Leu Thr His Asn Val Tyr Arg Gly Arg Met Val Tyr Trp Ala
                   165                 170                 175
   Asn Phe Tyr Glu Lys Leu Phe Asn Phe Arg Glu Ala Arg Tyr Phe Asp
               180                 185                 190
   Ile Lys Gly Glu Tyr Thr Gly Leu Thr Ser Lys Ala Met Ser Ala Pro
           195                 200                 205
   Asp Gly Met Ile Arg Ile Pro Leu Asn Glu Glu Ser Ser Lys Gly Ala
       210                 215                 220
   Gly Gln Ile Glu Glu Phe Leu Met Gln Phe Asn Gly Glu Gly Ile Gln
   225                 230                 235                 240
   His Val Ala Phe Leu Thr Asp Asp Leu Val Lys Thr Trp Asp Ala Leu
                   245                 250                 255
   Lys Lys Ile Gly Met Arg Phe Met Thr Ala Pro Pro Asp Thr Tyr Tyr
               260                 265                 270
   Glu Met Leu Glu Gly Arg Leu Pro Asp His Gly Glu Pro Val Asp Gln
           275                 280                 285
   Leu Gln Ala Arg Gly Ile Leu Leu Asp Gly Ser Ser Val Glu Gly Asp
       290                 295                 300
   Lys Arg Leu Leu Leu Gln Ile Phe Ser Glu Thr Leu Met Gly Pro Val
   305                 310                 315                 320
   Phe Phe Glu Phe Ile Gln Arg Lys Gly Asp Asp Gly Phe Gly Glu Gly
                   325                 330                 335
   Asn Phe Lys Ala Leu Phe Glu Ser Ile Glu Arg Asp Gln Val Arg Arg
               340                 345                 350
   Gly Val Leu Thr Ala Asp
           355
<210> 3
   <211> 1338
   <212> DNA
   <213> Arabidopsis thaliana
<220>
   <221> CDS
   <222> (1)..(1338)
<400> 3
   atg ggc cac caa aac gcc gcc gtt tca gag aat caa aac cat gat gac        48
   Met Gly His Gln Asn Ala Ala Val Ser Glu Asn Gln Asn His Asp Asp
   1               5                   10                  15
   ggc gct gcg tcg tcg ccg gga ttc aag ctc gtc gga ttt tcc aag ttc        96
   Gly Ala Ala Ser Ser Pro Gly Phe Lys Leu Val Gly Phe Ser Lys Phe
               20                  25                  30
   gta aga aag aat cca aag tct gat aaa ttc aag gtt aag cgc ttc cat       144
   Val Arg Lys Asn Pro Lys Ser Asp Lys Phe Lys Val Lys Arg Phe His
           35                  40                  45
   cac atc gag ttc tgg tgc ggc gac gca acc aac gtc gct cgt cgc ttc       192
   His Ile Glu Phe Trp Cys Gly Asp Ala Thr Asn Val Ala Arg Arg Phe
       50                  55                  60
   tcc tgg ggt ctg ggg atg aga ttc tcc gcc aaa tcc gat ctt tcc acc       240
   Ser Trp Gly Leu Gly Met Arg Phe Ser Ala Lys Ser Asp Leu Ser Thr
   65                  70                  75                  80
   gga aac atg gtt cac gcc tct tac cta ctc acc tcc ggt gac ctc cga       288
   Gly Asn Met Val His Ala Ser Tyr Leu Leu Thr Ser Gly Asp Leu Arg
                   85                  90                  95
   ttc ctt ttc act gct cct tac tct ccg tct ctc tcc gcc gga gag att       336
   Phe Leu Phe Thr Ala Pro Tyr Ser Pro Ser Leu Ser Ala Gly Glu Ile
               100                 105                 110
   aaa ccg aca acc aca gct tct atc cca agt ttc gat cac ggc tct tgt       384
   Lys Pro Thr Thr Thr Ala Ser Ile Pro Ser Phe Asp His Gly Ser Cys
           115                 120                 125
   cgt tcc ttc ttc tct tca cat ggt ctc ggt gtt aga gcc gtt gcg att       432
   Arg Ser Phe Phe Ser Ser His Gly Leu Gly Val Arg Ala Val Ala Ile
       130                 135                 140
   gaa gta gaa gac gca gag tca gct ttc tcc atc agt gta gct aat ggc       480
   Glu Val Glu Asp Ala Glu Ser Ala Phe Ser Ile Ser Val Ala Asn Gly
   145                 150                 155                 160
   gct att cct tcg tcg cct cct atc gtc ctc aat gaa gca gtt acg atc       528
   Ala Ile Pro Ser Ser Pro Pro Ile Val Leu Asn Glu Ala Val Thr Ile
                   165                 170                 175
   gct gag gtt aaa cta tac ggc gat gtt gtt ctc cga tat gtt agt tac       576
   Ala Glu Val Lys Leu Tyr Gly Asp Val Val Leu Arg Tyr Val Ser Tyr
               180                 185                 190
   aaa gca gaa gat acc gaa aaa tcc gaa ttc ttg cca ggg ttc gag cgt       624
   Lys Ala Glu Asp Thr Glu Lys Ser Glu Phe Leu Pro Gly Phe Glu Arg
           195                 200                 205
   gta gag gat gcg tcg tcg ttc cca ttg gat tat ggt atc cgg cgg ctt       672
   Val Glu Asp Ala Ser Ser Phe Pro Leu Asp Tyr Gly Ile Arg Arg Leu
       210                 215                 220
   gac cac gcc gtg gga aac gtt cct gag ctt ggt ccg gct tta act tat       720
   Asp His Ala Val Gly Asn Val Pro Glu Leu Gly Pro Ala Leu Thr Tyr
   225                 230                 235                 240
   gta gcg ggg ttc act ggt ttt cac caa ttc gca gag ttc aca gca gac       768
   Val Ala Gly Phe Thr Gly Phe His Gln Phe Ala Glu Phe Thr Ala Asp
                   245                 250                 255
   gac gtt gga acc gcc gag agc ggt tta aat tca gcg gtc ctg gct agc       816
   Asp Val Gly Thr Ala Glu Ser Gly Leu Asn Ser Ala Val Leu Ala Ser
               260                 265                 270
   aat gat gaa atg gtt ctt cta ccg att aac gag cca gtg cac gga aca       864
   Asn Asp Glu Met Val Leu Leu Pro Ile Asn Glu Pro Val His Gly Thr
           275                 280                 285
   aag agg aag agt cag att cag acg tat ttg gaa cat aac gaa ggc gca       912
   Lys Arg Lys Ser Gln Ile Gln Thr Tyr Leu Glu His Asn Glu Gly Ala
       290                 295                 300
   ggg cta caa cat ctg gct ctg atg agt gaa gac ata ttc agg acc ctg       960
   Gly Leu Gln His Leu Ala Leu Met Ser Glu Asp Ile Phe Arg Thr Leu
   305                 310                 315                 320
   aga gag atg agg aag agg agc agt att gga gga ttc gac ttc atg cct      1008
   Arg Glu Met Arg Lys Arg Ser Ser Ile Gly Gly Phe Asp Phe Met Pro
                   325                 330                 335
   tct cct ccg cct act tac tac cag aat ctc aag aaa cgg gtc ggc gac      1056
   Ser Pro Pro Pro Thr Tyr Tyr Gln Asn Leu Lys Lys Arg Val Gly Asp
               340                 345                 350
   gtg ctc agc gat gat cag atc aag gag tgt gag gaa tta ggg att ctt      1104
   Val Leu Ser Asp Asp Gln Ile Lys Glu Cys Glu Glu Leu Gly Ile Leu
           355                 360                 365
   gta gac aga gat gat caa ggg acg ttg ctt caa atc ttc aca aaa cca      1152
   Val Asp Arg Asp Asp Gln Gly Thr Leu Leu Gln Ile Phe Thr Lys Pro
       370                 375                 380
   cta ggt gac agg ccg acg ata ttt ata gag ata atc cag aga gta gga      1200
   Leu Gly Asp Arg Pro Thr Ile Phe Ile Glu Ile Ile Gln Arg Val Gly
   385                 390                 395                 400
   tgc atg atg aaa gat gag gaa ggg aag gct tac cag agt gga gga tgt      1248
   Cys Met Met Lys Asp Glu Glu Gly Lys Ala Tyr Gln Ser Gly Gly Cys
                   405                 410                 415
   ggt ggt ttt ggc aaa ggc aat ttc tct gag ctc ttc aag tcc att gaa      1296
   Gly Gly Phe Gly Lys Gly Asn Phe Ser Glu Leu Phe Lys Ser Ile Glu
               420                 425                 430
   gaa tac gaa aag act ctt gaa gcc aaa cag tta gtg gga tga              1338
   Glu Tyr Glu Lys Thr Leu Glu Ala Lys Gln Leu Val Gly
           435                 440                 445
<210> 4
   <211> 445
   <212> PRT
   <213> Arabidopsis thaliana
<400> 4
   Met Gly His Gln Asn Ala Ala Val Ser Glu Asn Gln Asn His Asp Asp
   1               5                   10                  15
   Gly Ala Ala Ser Ser Pro Gly Phe Lys Leu Val Gly Phe Ser Lys Phe
               20                  25                  30
   Val Arg Lys Asn Pro Lys Ser Asp Lys Phe Lys Val Lys Arg Phe His
           35                  40                  45
   His Ile Glu Phe Trp Cys Gly Asp Ala Thr Asn Val Ala Arg Arg Phe
       50                  55                  60
   Ser Trp Gly Leu Gly Met Arg Phe Ser Ala Lys Ser Asp Leu Ser Thr
   65                  70                  75                  80
   Gly Asn Met Val His Ala Ser Tyr Leu Leu Thr Ser Gly Asp Leu Arg
                   85                  90                  95
   Phe Leu Phe Thr Ala Pro Tyr Ser Pro Ser Leu Ser Ala Gly Glu Ile
               100                 105                 110
   Lys Pro Thr Thr Thr Ala Ser Ile Pro Ser Phe Asp His Gly Ser Cys
           115                 120                 125
   Arg Ser Phe Phe Ser Ser His Gly Leu Gly Val Arg Ala Val Ala Ile
       130                 135                 140
   Glu Val Glu Asp Ala Glu Ser Ala Phe Ser Ile Ser Val Ala Asn Gly
   145                 150                 155                 160
   Ala Ile Pro Ser Ser Pro Pro Ile Val Leu Asn Glu Ala Val Thr Ile
                   165                 170                 175
   Ala Glu Val Lys Leu Tyr Gly Asp Val Val Leu Arg Tyr Val Ser Tyr
               180                 185                 190
   Lys Ala Glu Asp Thr Glu Lys Ser Glu Phe Leu Pro Gly Phe Glu Arg
           195                 200                 205
   Val Glu Asp Ala Ser Ser Phe Pro Leu Asp Tyr Gly Ile Arg Arg Leu
       210                 215                 220
   Asp His Ala Val Gly Asn Val Pro Glu Leu Gly Pro Ala Leu Thr Tyr
   225                 230                 235                 240
   Val Ala Gly Phe Thr Gly Phe His Gln Phe Ala Glu Phe Thr Ala Asp
                   245                 250                 255
   Asp Val Gly Thr Ala Glu Ser Gly Leu Asn Ser Ala Val Leu Ala Ser
               260                 265                 270
   Asn Asp Glu Met Val Leu Leu Pro Ile Asn Glu Pro Val His Gly Thr
           275                 280                 285
   Lys Arg Lys Ser Gln Ile Gln Thr Tyr Leu Glu His Asn Glu Gly Ala
       290                 295                 300
   Gly Leu Gln His Leu Ala Leu Met Ser Glu Asp Ile Phe Arg Thr Leu
   305                 310                 315                 320
   Arg Glu Met Arg Lys Arg Ser Ser Ile Gly Gly Phe Asp Phe Met Pro
                   325                 330                 335
   Ser Pro Pro Pro Thr Tyr Tyr Gln Asn Leu Lys Lys Arg Val Gly Asp
               340                 345                 350
   Val Leu Ser Asp Asp Gln Ile Lys Glu Cys Glu Glu Leu Gly Ile Leu
           355                 360                 365
   Val Asp Arg Asp Asp Gln Gly Thr Leu Leu Gln Ile Phe Thr Lys Pro
   370 375 380
   Leu Gly Asp Arg Pro Thr Ile Phe Ile Glu Ile Ile Gln Arg Val Gly
   385 390 395 400
   Cys Met Met Lys Asp Glu Glu Gly Lys Ala Tyr Gln Ser Gly Gly Cys
   405 410 415
   Gly Gly Phe Gly Lys Gly Asn Phe Ser Glu Leu Phe Lys Ser Ile Glu
   420 425 430
   Glu Tyr Glu Lys Thr Leu Glu Ala Lys Gln Leu Val Gly
   435 440 445
<210> 5
   <211> 1182
   <212> DNA
   <213> Mus musculus
<220>
   <221> CDS
   <222> (1)..(1182)
<400> 5
   atg aca acc tac aac aac aaa gga cca aag cct gag aga ggc cgg ttc          48
   Met Thr Thr Tyr Asn Asn Lys Gly Pro Lys Pro Glu Arg Gly Arg Phe
   1               5                   10                  15
   ctc cat ttc cac tcg gtg acc ttc tgg gtt ggc aat gcc aag cag gct          96
   Leu His Phe His Ser Val Thr Phe Trp Val Gly Asn Ala Lys Gln Ala
               20                  25                  30
   gct tcc ttc tac tgc aac aag atg ggc ttt gaa cct ctg gcc tac agg         144
   Ala Ser Phe Tyr Cys Asn Lys Met Gly Phe Glu Pro Leu Ala Tyr Arg
           35                  40                  45
   ggc cta gag act ggc tcc cgg gag gta gtc agc cac gtc atc aag caa         192
   Gly Leu Glu Thr Gly Ser Arg Glu Val Val Ser His Val Ile Lys Gln
       50                  55                  60
   ggg aaa att gtg ttt gtt ctc tgc tct gct ctc aat ccc tgg aac aaa         240
   Gly Lys Ile Val Phe Val Leu Cys Ser Ala Leu Asn Pro Trp Asn Lys
   65                  70                  75                  80
   gag atg ggc gac cac ttg gtg aag cat ggc gac ggg gtg aaa gac atc         288
   Glu Met Gly Asp His Leu Val Lys His Gly Asp Gly Val Lys Asp Ile
                   85                  90                  95
   gca ttc gag gtg gaa gac tgc gac cac att gtg cag aaa gct cga gaa         336
   Ala Phe Glu Val Glu Asp Cys Asp His Ile Val Gln Lys Ala Arg Glu
               100                 105                 110
   cgg ggc gcc aaa att gtg cgg gag cca tgg gtg gag caa gac aaa ttt         384
   Arg Gly Ala Lys Ile Val Arg Glu Pro Trp Val Glu Gln Asp Lys Phe
           115                 120                 125
   ggg aag gtg aag ttt gct gtg ctg cag acg tat gga gat acc aca cac         432
   Gly Lys Val Lys Phe Ala Val Leu Gln Thr Tyr Gly Asp Thr Thr His
       130                 135                 140
   acc ctg gtg gag aag atc aac tac act ggc cgt ttc tta cct gga ttc      480
   Thr Leu Val Glu Lys Ile Asn Tyr Thr Gly Arg Phe Leu Pro Gly Phe
   145                 150                 155                 160
   gag gcc cca aca tac aag gat acc ctg ctt cca aaa cta ccc aga tgt      528
   Glu Ala Pro Thr Tyr Lys Asp Thr Leu Leu Pro Lys Leu Pro Arg Cys
                   165                 170                 175
   aac ctt gag atc att gac cac att gta ggc aac caa ccc gac caa gaa      576
   Asn Leu Glu Ile Ile Asp His Ile Val Gly Asn Gln Pro Asp Gln Glu
               180                 185                 190
   atg cag tct gcc tca gaa tgg tac ctg aaa aac ctg cag ttc cac cgg      624
   Met Gln Ser Ala Ser Glu Trp Tyr Leu Lys Asn Leu Gln Phe His Arg
           195                 200                 205
   ttc tgg tcc gtg gac gac acg cag gtg cac acg gag tac agc tct ctg      672
   Phe Trp Ser Val Asp Asp Thr Gln Val His Thr Glu Tyr Ser Ser Leu
       210                 215                 220
   cgc tcc att gtg gtg acc aac tac gag gaa tcc atc aaa atg ccc atc      720
   Arg Ser Ile Val Val Thr Asn Tyr Glu Glu Ser Ile Lys Met Pro Ile
   225                 230                 235                 240
   aac gag cca gct ccg ggc agg aag aag tct cag atc cag gaa tat gtg      768
   Asn Glu Pro Ala Pro Gly Arg Lys Lys Ser Gln Ile Gln Glu Tyr Val
                   245                 250                 255
   gac tat aat ggg ggt gct ggg gtc cag cac atc gct ctc aag acg gaa      816
   Asp Tyr Asn Gly Gly Ala Gly Val Gln His Ile Ala Leu Lys Thr Glu
               260                 265                 270
   gac atc atc aca gca atc cgc cac ttg agg gag cga ggc acg gag ttc      864
   Asp Ile Ile Thr Ala Ile Arg His Leu Arg Glu Arg Gly Thr Glu Phe
           275                 280                 285
   ttg gcc gcc cca tct tct tac tac aaa ctg ctt cgg gag aat ctc aag      912
   Leu Ala Ala Pro Ser Ser Tyr Tyr Lys Leu Leu Arg Glu Asn Leu Lys
       290                 295                 300
   tca gcc aag atc cag gtg aaa gag agc atg gac gtc ctg gag gag ctg      960
   Ser Ala Lys Ile Gln Val Lys Glu Ser Met Asp Val Leu Glu Glu Leu
   305                 310                 315                 320
   cat atc cta gtc gac tat gac gag aaa ggc tac ctc cta cag atc ttc     1008
   His Ile Leu Val Asp Tyr Asp Glu Lys Gly Tyr Leu Leu Gln Ile Phe
                   325                 330                 335
   acc aag ccc atg cag gac cgg ccc aca ctc ttc ctg gaa gtc att caa     1056
   Thr Lys Pro Met Gln Asp Arg Pro Thr Leu Phe Leu Glu Val Ile Gln
               340                 345                 350
   cgt cac aac cac cag ggc ttt gga gcg ggc aac ttc aac tct ctg ttc     1104
   Arg His Asn His Gln Gly Phe Gly Ala Gly Asn Phe Asn Ser Leu Phe
           355                 360                 365
   aag gcg ttc gag gag gag caa gcc cta cgg ggc aac ctc act gac ctg     1152
   Lys Ala Phe Glu Glu Glu Gln Ala Leu Arg Gly Asn Leu Thr Asp Leu
       370                 375                 380
   gag ccc aat ggt gtg agg tct gga atg taa                         1182
   Glu Pro Asn Gly Val Arg Ser Gly Met
   385                 390
<210> 6
   <211> 393
   <212> PRT
   <213> Mus musculus
<400> 6
   Met Thr Thr Tyr Asn Asn Lys Gly Pro Lys Pro Glu Arg Gly Arg Phe
   1               5                   10                  15
   Leu His Phe His Ser Val Thr Phe Trp Val Gly Asn Ala Lys Gln Ala
               20                  25                  30
   Ala Ser Phe Tyr Cys Asn Lys Met Gly Phe Glu Pro Leu Ala Tyr Arg
           35                  40                  45
   Gly Leu Glu Thr Gly Ser Arg Glu Val Val Ser His Val Ile Lys Gln
       50                  55                  60
   Gly Lys Ile Val Phe Val Leu Cys Ser Ala Leu Asn Pro Trp Asn Lys
   65                  70                  75                  80
   Glu Met Gly Asp His Leu Val Lys His Gly Asp Gly Val Lys Asp Ile
                   85                  90                  95
   Ala Phe Glu Val Glu Asp Cys Asp His Ile Val Gln Lys Ala Arg Glu
               100                 105                 110
   Arg Gly Ala Lys Ile Val Arg Glu Pro Trp Val Glu Gln Asp Lys Phe
           115                 120                 125
   Gly Lys Val Lys Phe Ala Val Leu Gln Thr Tyr Gly Asp Thr Thr His
       130                 135                 140
   Thr Leu Val Glu Lys Ile Asn Tyr Thr Gly Arg Phe Leu Pro Gly Phe
   145                 150                 155                 160
   Glu Ala Pro Thr Tyr Lys Asp Thr Leu Leu Pro Lys Leu Pro Arg Cys
                   165                 170                 175
   Asn Leu Glu Ile Ile Asp His Ile Val Gly Asn Gln Pro Asp Gln Glu
               180                 185                 190
   Met Gln Ser Ala Ser Glu Trp Tyr Leu Lys Asn Leu Gln Phe His Arg
           195                 200                 205
   Phe Trp Ser Val Asp Asp Thr Gln Val His Thr Glu Tyr Ser Ser Leu
       210                 215                 220
   Arg Ser Ile Val Val Thr Asn Tyr Glu Glu Ser Ile Lys Met Pro Ile
   225                 230                 235                 240
   Asn Glu Pro Ala Pro Gly Arg Lys Lys Ser Gln Ile Gln Glu Tyr Val
                   245                 250                 255
   Asp Tyr Asn Gly Gly Ala Gly Val Gln His Ile Ala Leu Lys Thr Glu
               260                 265                 270
   Asp Ile Ile Thr Ala Ile Arg His Leu Arg Glu Arg Gly Thr Glu Phe
           275                 280                 285
   Leu Ala Ala Pro Ser Ser Tyr Tyr Lys Leu Leu Arg Glu Asn Leu Lys
       290                 295                 300
   Ser Ala Lys Ile Gln Val Lys Glu Ser Met Asp Val Leu Glu Glu Leu
   305                 310                 315                 320
   His Ile Leu Val Asp Tyr Asp Glu Lys Gly Tyr Leu Leu Gln Ile Phe
                   325                 330                 335
   Thr Lys Pro Met Gln Asp Arg Pro Thr Leu Phe Leu Glu Val Ile Gln
               340                 345                 350
   Arg His Asn His Gln Gly Phe Gly Ala Gly Asn Phe Asn Ser Leu Phe
           355                 360                 365
   Lys Ala Phe Glu Glu Glu Gln Ala Leu Arg Gly Asn Leu Thr Asp Leu
       370                 375                 380
   Glu Pro Asn Gly Val Arg Ser Gly Met
   385                 390
<210> 7
   <211> 1200
   <212> DNA
   <213> Coccidioides immitis
<220>
   <221> CDS
   <222> (1)..(1200)
<400> 7
   atg gca cca gcc gct gac tcc ccg acg ctt caa ccc gcc cag ccc tct       48
   Met Ala Pro Ala Ala Asp Ser Pro Thr Leu Gln Pro Ala Gln Pro Ser
   1               5                   10                  15
   gat ctc aat cag tat aga gga tac gac cac gtc cac tgg tat gtc gga        96
   Asp Leu Asn Gln Tyr Arg Gly Tyr Asp His Val His Trp Tyr Val Gly
               20                  25                  30
   aac gct aag cag gcc gct acc tac tat gtc act cgc atg ggt ttc gag       144
   Asn Ala Lys Gln Ala Ala Thr Tyr Tyr Val Thr Arg Met Gly Phe Glu
           35                  40                  45
   aga gta gcc tat cgc gga ttg gag act ggc tcc aaa gcg gtg gcc tcg       192
   Arg Val Ala Tyr Arg Gly Leu Glu Thr Gly Ser Lys Ala Val Ala Ser
       50                  55                  60
   cat gtt gtg cga aac gga aac atc acc ttc atc ttg act tcg ccc ctt       240
   His Val Val Arg Asn Gly Asn Ile Thr Phe Ile Leu Thr Ser Pro Leu
   65                  70                  75                  80
   cga tcc gtt gag cag gct tct cgt ttc ccc gag gac gag gct ctc ctg       288
   Arg Ser Val Glu Gln Ala Ser Arg Phe Pro Glu Asp Glu Ala Leu Leu
                   85                  90                  95
   aag gag atc cac gcc cat ctc gag aga cac ggc gat ggt gtc aag gac       336
   Lys Glu Ile His Ala His Leu Glu Arg His Gly Asp Gly Val Lys Asp
               100                 105                 110
   gtc gcc ttc gag gtc gac tgc gta gag tct gtc ttc tcg gct gcc gtt       384
   Val Ala Phe Glu Val Asp Cys Val Glu Ser Val Phe Ser Ala Ala Val
           115                 120                 125
   agg aac ggt gct gag gtt gtt tcc gat gtc aga acg gtt gaa gat gag       432
   Arg Asn Gly Ala Glu Val Val Ser Asp Val Arg Thr Val Glu Asp Glu
       130                 135                 140
   gat ggc cag atc aag atg gcg acc atc cga act tat ggc gag acc act       480
   Asp Gly Gln Ile Lys Met Ala Thr Ile Arg Thr Tyr Gly Glu Thr Thr
   145                 150                 155                 160
   cac acc ctc atc gaa aga tcc ggc tac agg ggc gga ttc atg ccg gga       528
   His Thr Leu Ile Glu Arg Ser Gly Tyr Arg Gly Gly Phe Met Pro Gly
                   165                 170                 175
   tac cgg atg gag agc aat gcc gac gcc act tcc aag ttc ctt cca aag       576
   Tyr Arg Met Glu Ser Asn Ala Asp Ala Thr Ser Lys Phe Leu Pro Lys
               180                 185                 190
   gtt gtg ctt gag aga ata gac cac tgc gtt gga aac cag gac tgg gac       624
   Val Val Leu Glu Arg Ile Asp His Cys Val Gly Asn Gln Asp Trp Asp
           195                 200                 205
   gag atg gag cga gtc tgc gac tac tac gag aag atc ctc gga ttc cac       672
   Glu Met Glu Arg Val Cys Asp Tyr Tyr Glu Lys Ile Leu Gly Phe His
       210                 215                 220
   cgt ttc tgg tcc gtt gat gac aag gac atc tgc act gaa ttc tct gca       720
   Arg Phe Trp Ser Val Asp Asp Lys Asp Ile Cys Thr Glu Phe Ser Ala
   225                 230                 235                 240
   ctg aag agt atc gtc atg gca tct cca aat gat atc gtc aag atg ccc       768
   Leu Lys Ser Ile Val Met Ala Ser Pro Asn Asp Ile Val Lys Met Pro
                   245                 250                 255
   atc aac gag ccc gcc aag gga aag aaa caa tcc cag att gaa gaa tat       816
   Ile Asn Glu Pro Ala Lys Gly Lys Lys Gln Ser Gln Ile Glu Glu Tyr
                   260                 265                 270
   gtt gac ttc tac aat ggt gct ggc gtt cag cac att gct ctc cga acc        864
   Val Asp Phe Tyr Asn Gly Ala Gly Val Gln His Ile Ala Leu Arg Thr
               275                 280                 285
   aac aac atc atc gat gcc atc acc aac ctc aag gcg cgc ggc acc gaa        912
   Asn Asn Ile Ile Asp Ala Ile Thr Asn Leu Lys Ala Arg Gly Thr Glu
       290                 295                 300
   ttc atc aag gtt cca gag acc tac tat gaa gac atg aag att cgc ctc        960
   Phe Ile Lys Val Pro Glu Thr Tyr Tyr Glu Asp Met Lys Ile Arg Leu
   305                 310                 315                 320
   aag aga caa ggc ctg gtc ctc gat gag gac ttt gag acc ctg aag agc       1008
   Lys Arg Gln Gly Leu Val Leu Asp Glu Asp Phe Glu Thr Leu Lys Ser
                   325                 330                 335
   ctg gac atc ctt atc gac ttt gac gag aat ggg tat ctc ctg cag ctt       1056
   Leu Asp Ile Leu Ile Asp Phe Asp Glu Asn Gly Tyr Leu Leu Gln Leu
                   340                 345                 350
   ttc acc aag cat ctc atg gat cgc cca acc gtt ttc att gaa atc atc       1104
   Phe Thr Lys His Leu Met Asp Arg Pro Thr Val Phe Ile Glu Ile Ile
               355                 360                 365
   caa cgc aac aac ttt tcc ggt ttc ggt gcg ggc aac ttc agg gcc ctc       1152
   Gln Arg Asn Asn Phe Ser Gly Phe Gly Ala Gly Asn Phe Arg Ala Leu
       370                 375                 380
   ttc gag gct att gag cgt gag cag gct ctc cgt ggc acc ctt atc tag       1200
   Phe Glu Ala Ile Glu Arg Glu Gln Ala Leu Arg Gly Thr Leu Ile
   385                 390                 395
<210> 8
   <211> 399
   <212> PRT
   <213> Coccidioides immitis
<400> 8
   Met Ala Pro Ala Ala Asp Ser Pro Thr Leu Gln Pro Ala Gln Pro Ser
   1               5                   10                  15
   Asp Leu Asn Gln Tyr Arg Gly Tyr Asp His Val His Trp Tyr Val Gly
               20                  25                  30
   Asn Ala Lys Gln Ala Ala Thr Tyr Tyr Val Thr Arg Met Gly Phe Glu
           35                  40                  45
   Arg Val Ala Tyr Arg Gly Leu Glu Thr Gly Ser Lys Ala Val Ala Ser
       50                  55                  60
   His Val Val Arg Asn Gly Asn Ile Thr Phe Ile Leu Thr Ser Pro Leu
   65                  70                  75                  80
   Arg Ser Val Glu Gln Ala Ser Arg Phe Pro Glu Asp Glu Ala Leu Leu
                   85                  90                  95
   Lys Glu Ile His Ala His Leu Glu Arg His Gly Asp Gly Val Lys Asp
               100                 105                 110
   Val Ala Phe Glu Val Asp Cys Val Glu Ser Val Phe Ser Ala Ala Val
           115                 120                 125
   Arg Asn Gly Ala Glu Val Val Ser Asp Val Arg Thr Val Glu Asp Glu
       130                 135                 140
   Asp Gly Gln Ile Lys Met Ala Thr Ile Arg Thr Tyr Gly Glu Thr Thr
   145                 150                 155                 160
   His Thr Leu Ile Glu Arg Ser Gly Tyr Arg Gly Gly Phe Met Pro Gly
                   165                 170                 175
   Tyr Arg Met Glu Ser Asn Ala Asp Ala Thr Ser Lys Phe Leu Pro Lys
               180                 185                 190
   Val Val Leu Glu Arg Ile Asp His Cys Val Gly Asn Gln Asp Trp Asp
           195                 200                 205
   Glu Met Glu Arg Val Cys Asp Tyr Tyr Glu Lys Ile Leu Gly Phe His
       210                 215                 220
   Arg Phe Trp Ser Val Asp Asp Lys Asp Ile Cys Thr Glu Phe Ser Ala
   225                 230                 235                 240
   Leu Lys Ser Ile Val Met Ala Ser Pro Asn Asp Ile Val Lys Met Pro
                   245                 250                 255
   Ile Asn Glu Pro Ala Lys Gly Lys Lys Gln Ser Gln Ile Glu Glu Tyr
               260                 265                 270
   Val Asp Phe Tyr Asn Gly Ala Gly Val Gln His Ile Ala Leu Arg Thr
           275                 280                 285
   Asn Asn Ile Ile Asp Ala Ile Thr Asn Leu Lys Ala Arg Gly Thr Glu
       290                 295                 300
   Phe Ile Lys Val Pro Glu Thr Tyr Tyr Glu Asp Met Lys Ile Arg Leu
   305                 310                 315                 320
   Lys Arg Gln Gly Leu Val Leu Asp Glu Asp Phe Glu Thr Leu Lys Ser
                   325                 330                 335
   Leu Asp Ile Leu Ile Asp Phe Asp Glu Asn Gly Tyr Leu Leu Gln Leu
   340 345 350
   Phe Thr Lys His Leu Met Asp Arg Pro Thr Val Phe Ile Glu Ile Ile
   355 360 365
   Gln Arg Asn Asn Phe Ser Gly Phe Gly Ala Gly Asn Phe Arg Ala Leu
   370 375 380
   Phe Glu Ala Ile Glu Arg Glu Gln Ala Leu Arg Gly Thr Leu Ile
   385 390 395
<210> 9
   <211> 1260
   <212> DNA
   <213> Mycosphaerella graminicola
<220>
   <221> CDS
   <222> (1)..(1260)
<400> 9
   atg gca ccc gga gca ctc ctc gtc aca tca cag aat gga aga acg agc        48
   Met Ala Pro Gly Ala Leu Leu Val Thr Ser Gln Asn Gly Arg Thr Ser
   1               5                   10                  15
   ccc ctc tac gac tcc gat ggc tat gta cca gcg cct gcg gct cta gta        96
   Pro Leu Tyr Asp Ser Asp Gly Tyr Val Pro Ala Pro Ala Ala Leu Val
               20                  25                  30
   gta ggt ggt gag gtc aat tac aga ggc tac cat cat gca gaa tgg tgg       144
   Val Gly Gly Glu Val Asn Tyr Arg Gly Tyr His His Ala Glu Trp Trp
           35                  40                  45
   gtg ggc aat gca aag cag gtg gcg caa ttc tac atc aca cgc atg ggc       192
   Val Gly Asn Ala Lys Gln Val Ala Gln Phe Tyr Ile Thr Arg Met Gly
       50                  55                  60
   ttc gag cct gtt gca cac aaa ggt ctg gag acc gga tct cgc ttc ttt       240
   Phe Glu Pro Val Ala His Lys Gly Leu Glu Thr Gly Ser Arg Phe Phe
   65                  70                  75                  80
   gcc agc cac gtt gtc cag aac aac ggc gtt cgc ttc gtc ttc aca tca       288
   Ala Ser His Val Val Gln Asn Asn Gly Val Arg Phe Val Phe Thr Ser
                   85                  90                  95
   cct gtt cgg tca tcg gca cgg caa aca ctc aaa gca gcg cct ctc gcg       336
   Pro Val Arg Ser Ser Ala Arg Gln Thr Leu Lys Ala Ala Pro Leu Ala
               100                 105                 110
   gac caa gca cgc ctc gac gaa atg tac gat cac ctc gac aag cac gga       384
   Asp Gln Ala Arg Leu Asp Glu Met Tyr Asp His Leu Asp Lys His Gly
           115                 120                 125
   gat gga gtg aag gat gtt gcc ttc gaa gtt gac gat gtc ttg gct gtg       432
   Asp Gly Val Lys Asp Val Ala Phe Glu Val Asp Asp Val Leu Ala Val
       130                 135                 140
   tac gag aac gca gtt gcg aat ggt gcg gag tcc gtc agt tca cca cat      480
   Tyr Glu Asn Ala Val Ala Asn Gly Ala Glu Ser Val Ser Ser Pro His
   145                 150                 155                 160
   acc gat tca tgc gac gaa ggc gat gtg atc tcc gcg gcg atc aag aca      528
   Thr Asp Ser Cys Asp Glu Gly Asp Val Ile Ser Ala Ala Ile Lys Thr
                   165                 170                 175
   tac gga gac acc acg cac act ttc atc caa cgc aca aca tat aca gga      576
   Tyr Gly Asp Thr Thr His Thr Phe Ile Gln Arg Thr Thr Tyr Thr Gly
               180                 185                 190
   cca ttt ctt cct ggc tat cga tca tgt acc aca gtg gat tcg gcc aac      624
   Pro Phe Leu Pro Gly Tyr Arg Ser Cys Thr Thr Val Asp Ser Ala Asn
           195                 200                 205
   aag ttc ttg cca cca gtc aat ctc gaa gcg atc gat cac tgt gtc ggc      672
   Lys Phe Leu Pro Pro Val Asn Leu Glu Ala Ile Asp His Cys Val Gly
       210                 215                 220
   aat caa gac tgg gac gag atg agc gat gcc tgc gac ttc tac gag cgc      720
   Asn Gln Asp Trp Asp Glu Met Ser Asp Ala Cys Asp Phe Tyr Glu Arg
   225                 230                 235                 240
   tgt ctt gga ttc cat cgc ttc tgg agt gtc gat gac aag gac atc tgt      768
   Cys Leu Gly Phe His Arg Phe Trp Ser Val Asp Asp Lys Asp Ile Cys
                   245                 250                 255
   acg gag ttc tcc gcg ctg aag tct atc gtt atg agt tct ccc aac cag      816
   Thr Glu Phe Ser Ala Leu Lys Ser Ile Val Met Ser Ser Pro Asn Gln
               260                 265                 270
   gta gtc aag atg cca atc aac gag ccc gcc cat ggc aag aag aag agc      864
   Val Val Lys Met Pro Ile Asn Glu Pro Ala His Gly Lys Lys Lys Ser
           275                 280                 285
   cag atc gag gag tac gtc gat ttc tac aat gga cct ggc gta caa cac      912
   Gln Ile Glu Glu Tyr Val Asp Phe Tyr Asn Gly Pro Gly Val Gln His
       290                 295                 300
   atc gct ctc cgt acg cca aac atc atc gag gca gta tca aac ttg cgg      960
   Ile Ala Leu Arg Thr Pro Asn Ile Ile Glu Ala Val Ser Asn Leu Arg
   305                 310                 315                 320
   tca aga ggc gtg gag ttc atc agc gtg cca gat acg tac tac gag aac     1008
   Ser Arg Gly Val Glu Phe Ile Ser Val Pro Asp Thr Tyr Tyr Glu Asn
                   325                 330                 335
   atg cgt ctt cgt ctc aaa gcg gca gga atg aag ctg gag gag tca ttc     1056
   Met Arg Leu Arg Leu Lys Ala Ala Gly Met Lys Leu Glu Glu Ser Phe
               340                 345                 350
   gac atc att caa aag ctg aac atc ctc atc gat ttc gac gaa ggt ggc     1104
   Asp Ile Ile Gln Lys Leu Asn Ile Leu Ile Asp Phe Asp Glu Gly Gly
           355                 360                 365
   tat ttg ctg cag ctg ttc acg aag ccg ctg atg gat cgg ccg acg gtc     1152
   Tyr Leu Leu Gln Leu Phe Thr Lys Pro Leu Met Asp Arg Pro Thr Val
       370                 375                 380
   ttc att gaa atc att caa cgg aac aac ttt gat ggc ttc gga gct gga     1200
   Phe Ile Glu Ile Ile Gln Arg Asn Asn Phe Asp Gly Phe Gly Ala Gly
   385                 390                 395                 400
   aac ttc aag agt ctg ttc gag gcg att gag cga gag cag gac ttg cgt      1248
   Asn Phe Lys Ser Leu Phe Glu Ala Ile Glu Arg Glu Gln Asp Leu Arg
                   405                 410                 415
   ggc aat ctc tag                                                      1260
   Gly Asn Leu
<210> 10
   <211> 419
   <212> PRT
   <213> Mycosphaerella graminicola
<400> 10
   Met Ala Pro Gly Ala Leu Leu Val Thr Ser Gln Asn Gly Arg Thr Ser
   1               5                   10                  15
   Pro Leu Tyr Asp Ser Asp Gly Tyr Val Pro Ala Pro Ala Ala Leu Val
               20                  25                  30
   Val Gly Gly Glu Val Asn Tyr Arg Gly Tyr His His Ala Glu Trp Trp
           35                  40                  45
   Val Gly Asn Ala Lys Gln Val Ala Gln Phe Tyr Ile Thr Arg Met Gly
       50                  55                  60
   Phe Glu Pro Val Ala His Lys Gly Leu Glu Thr Gly Ser Arg Phe Phe
   65                  70                  75                  80
   Ala Ser His Val Val Gln Asn Asn Gly Val Arg Phe Val Phe Thr Ser
                   85                  90                  95
   Pro Val Arg Ser Ser Ala Arg Gln Thr Leu Lys Ala Ala Pro Leu Ala
               100                 105                 110
   Asp Gln Ala Arg Leu Asp Glu Met Tyr Asp His Leu Asp Lys His Gly
           115                 120                 125
   Asp Gly Val Lys Asp Val Ala Phe Glu Val Asp Asp Val Leu Ala Val
       130                 135                 140
   Tyr Glu Asn Ala Val Ala Asn Gly Ala Glu Ser Val Ser Ser Pro His
   145                 150                 155                 160
   Thr Asp Ser Cys Asp Glu Gly Asp Val Ile Ser Ala Ala Ile Lys Thr
                   165                 170                 175
   Tyr Gly Asp Thr Thr His Thr Phe Ile Gln Arg Thr Thr Tyr Thr Gly
               180                 185                 190
   Pro Phe Leu Pro Gly Tyr Arg Ser Cys Thr Thr Val Asp Ser Ala Asn
   195 200 205
   Lys Phe Leu Pro Pro Val Asn Leu Glu Ala Ile Asp His Cys Val Gly
   210 215 220
   Asn Gln Asp Trp Asp Glu Met Ser Asp Ala Cys Asp Phe Tyr Glu Arg
   225 230 235 240
   Cys Leu Gly Phe His Arg Phe Trp Ser Val Asp Asp Lys Asp Ile Cys
   245 250 255
   Thr Glu Phe Ser Ala Leu Lys Ser Ile Val Met Ser Ser Pro Asn Gln
   260 265 270
   Val Val Lys Met Pro Ile Asn Glu Pro Ala His Gly Lys Lys Lys Ser
   275 280 285
   Gln Ile Glu Glu Tyr Val Asp Phe Tyr Asn Gly Pro Gly Val Gln His
   290 295 300
   Ile Ala Leu Arg Thr Pro Asn Ile Ile Glu Ala Val Ser Asn Leu Arg
   305 310 315 320
   Ser Arg Gly Val Glu Phe Ile Ser Val Pro Asp Thr Tyr Tyr Glu Asn
   325 330 335
   Met Arg Leu Arg Leu Lys Ala Ala Gly Met Lys Leu Glu Glu Ser Phe
   340 345 350
   Asp Ile Ile Gln Lys Leu Asn Ile Leu Ile Asp Phe Asp Glu Gly Gly
   355 360 365
   Tyr Leu Leu Gln Leu Phe Thr Lys Pro Leu Met Asp Arg Pro Thr Val
   370 375 380
   Phe Ile Glu Ile Ile Gln Arg Asn Asn Phe Asp Gly Phe Gly Ala Gly
   385 390 395 400
   Asn Phe Lys Ser Leu Phe Glu Ala Ile Glu Arg Glu Gln Asp Leu Arg
   405 410 415
   Gly Asn Leu
<210> 11
   <211> 1305
   <212> DNA
   <213> Hordeum vulgare
<220>
   <221> CDS
   <222> (1)..(1305)
<400> 11
   atg ccg ccc acc ccc acc acc ccc gcg gct acc ggc gcc gcc gcc gcg       48
   Met Pro Pro Thr Pro Thr Thr Pro Ala Ala Thr Gly Ala Ala Ala Ala
   1               5                   10                  15
   gtg acg ccg gag cac gcg cga ccg cac cga atg gtc cgc ttc aac ccg       96
   Val Thr Pro Glu His Ala Arg Pro His Arg Met Val Arg Phe Asn Pro
               20                  25                  30
   cgc agc gac cgc ttc cac acg ctc tcc ttc cac cac gtc gag ttc tgg      144
   Arg Ser Asp Arg Phe His Thr Leu Ser Phe His His Val Glu Phe Trp
           35                  40                  45
   tgc gcg gac gcc gcc tcc gcc gcc ggc cgc ttc gcg ttc gcg ctc ggc      192
   Cys Ala Asp Ala Ala Ser Ala Ala Gly Arg Phe Ala Phe Ala Leu Gly
       50                  55                  60
   gcg ccg ctc gcc gcc agg tcc gac ctc tcc acg ggg aac tcc gcg cac      240
   Ala Pro Leu Ala Ala Arg Ser Asp Leu Ser Thr Gly Asn Ser Ala His
   65                  70                  75                  80
   gcc tcc cag ctg ctc cgc tcg ggc tcc ctc gcc ttc ctc ttc acc gcg      288
   Ala Ser Gln Leu Leu Arg Ser Gly Ser Leu Ala Phe Leu Phe Thr Ala
                   85                  90                  95
   ccc tac gcc aac ggc tgc gac gcc gcc acc gcc tcc ctg ccc tcc ttc      336
   Pro Tyr Ala Asn Gly Cys Asp Ala Ala Thr Ala Ser Leu Pro Ser Phe
               100                 105                 110
   tcc gcc gac gcc gcg cgc cgg ttc tcc gcc gac cac ggg atc gcg gtg      384
   Ser Ala Asp Ala Ala Arg Arg Phe Ser Ala Asp His Gly Ile Ala Val
           115                 120                 125
   cgc tcc gta gcg ctg cgc gtc gca gac gcc gcc gag gcc ttc cgc gcc      432
   Arg Ser Val Ala Leu Arg Val Ala Asp Ala Ala Glu Ala Phe Arg Ala
       130                 135                 140
   agt cgt cga cgg ggc gcg cgc ccg gcc ttc gcc ccc gtg gac ctc ggc      480
   Ser Arg Arg Arg Gly Ala Arg Pro Ala Phe Ala Pro Val Asp Leu Gly
   145                 150                 155                 160
   cgc ggc ttc gcg ttc gcg gag gtc gag ctc tac ggc gac gtc gtg ctc      528
   Arg Gly Phe Ala Phe Ala Glu Val Glu Leu Tyr Gly Asp Val Val Leu
                   165                 170                 175
   cgc ttc gtc agc cac ccg gac ggc acg gac gtg ccc ttc ttg ccg ggg      576
   Arg Phe Val Ser His Pro Asp Gly Thr Asp Val Pro Phe Leu Pro Gly
               180                 185                 190
   ttc gag ggc gta acc aac ccg gac gcc gtg gac tac ggc ctg acg cgg      624
   Phe Glu Gly Val Thr Asn Pro Asp Ala Val Asp Tyr Gly Leu Thr Arg
           195                 200                 205
   ttc gac cac gtc gtc ggc aac gtc ccg gag ctt gcc ccc gcc gca gcc      672
   Phe Asp His Val Val Gly Asn Val Pro Glu Leu Ala Pro Ala Ala Ala
       210                 215                 220
   tac atc gcc ggg ttc acg ggg ttc cac gag ttc gcc gag ttc acg gcg      720
   Tyr Ile Ala Gly Phe Thr Gly Phe His Glu Phe Ala Glu Phe Thr Ala
   225                 230                 235                 240
   gag gac gtg ggc acg acc gag agc ggg ctc aac tcg gtg gtg ctc gcc      768
   Glu Asp Val Gly Thr Thr Glu Ser Gly Leu Asn Ser Val Val Leu Ala
                   245                 250                 255
   aac aac tcg gag ggc gtg ctg ctg ccg ctc aac gag ccg gtg cac ggc      816
   Asn Asn Ser Glu Gly Val Leu Leu Pro Leu Asn Glu Pro Val His Gly
               260                 265                 270
   acc aag cgc cgg agc cag ata cag acg ttc ctg gaa cac cac ggc ggc      864
   Thr Lys Arg Arg Ser Gln Ile Gln Thr Phe Leu Glu His His Gly Gly
           275                 280                 285
   ccg ggc gtg cag cac atc gcg gtg gcc agc agt gac gtg ctc agg acg      912
   Pro Gly Val Gln His Ile Ala Val Ala Ser Ser Asp Val Leu Arg Thr
       290                 295                 300
   ctc agg aag atg cgt gcg cgc tcc gcc atg ggc ggc ttc gac ttc ctg      960
   Leu Arg Lys Met Arg Ala Arg Ser Ala Met Gly Gly Phe Asp Phe Leu
   305                 310                 315                 320
   cca ccc ccg ctg ccg aag tac tac gaa ggc gtg cga cgc ctt gcc ggg     1008
   Pro Pro Pro Leu Pro Lys Tyr Tyr Glu Gly Val Arg Arg Leu Ala Gly
                   325                 330                 335
   gat gtc ctc tcg gag gcg cag atc aag gaa tgc cag gag ctg ggt gtg     1056
   Asp Val Leu Ser Glu Ala Gln Ile Lys Glu Cys Gln Glu Leu Gly Val
               340                 345                 350
   ctc gtc gat agg gac gac caa ggg gtg ttg ctc caa atc ttc acc aag     1104
   Leu Val Asp Arg Asp Asp Gln Gly Val Leu Leu Gln Ile Phe Thr Lys
           355                 360                 365
   cca gta ggg gac agg ccg acc ttg ttc ctg gag atg atc cag agg atc     1152
   Pro Val Gly Asp Arg Pro Thr Leu Phe Leu Glu Met Ile Gln Arg Ile
       370                 375                 380
   ggg tgc atg gag aag gac gag aga ggg gaa gag tac cag aag ggt ggc     1200
   Gly Cys Met Glu Lys Asp Glu Arg Gly Glu Glu Tyr Gln Lys Gly Gly
   385                 390                 395                 400
   tgc ggc ggg ttc ggc aaa ggc aac ttc tcc gag ctg ttc aag tcc att     1248
   Cys Gly Gly Phe Gly Lys Gly Asn Phe Ser Glu Leu Phe Lys Ser Ile
                   405                 410                 415
   gaa gat tac gag aag tcc ctt gaa gcc aag caa tct gct gca gtt cag     1296
   Glu Asp Tyr Glu Lys Ser Leu Glu Ala Lys Gln Ser Ala Ala Val Gln
               420                 425                 430
   gga tca tag                                                         1305
   Gly Ser
<210> 12
   <211> 434
   <212> PRT
   <213> Hordeum vulgare
<400> 12
   Met Pro Pro Thr Pro Thr Thr Pro Ala Ala Thr Gly Ala Ala Ala Ala
   1               5                   10                  15
   Val Thr Pro Glu His Ala Arg Pro His Arg Met Val Arg Phe Asn Pro
               20                  25                  30
   Arg Ser Asp Arg Phe His Thr Leu Ser Phe His His Val Glu Phe Trp
           35                  40                  45
   Cys Ala Asp Ala Ala Ser Ala Ala Gly Arg Phe Ala Phe Ala Leu Gly
       50                  55                  60
   Ala Pro Leu Ala Ala Arg Ser Asp Leu Ser Thr Gly Asn Ser Ala His
   65                  70                  75                  80
   Ala Ser Gln Leu Leu Arg Ser Gly Ser Leu Ala Phe Leu Phe Thr Ala
                   85                  90                  95
   Pro Tyr Ala Asn Gly Cys Asp Ala Ala Thr Ala Ser Leu Pro Ser Phe
               100                 105                 110
   Ser Ala Asp Ala Ala Arg Arg Phe Ser Ala Asp His Gly Ile Ala Val
           115                 120                 125
   Arg Ser Val Ala Leu Arg Val Ala Asp Ala Ala Glu Ala Phe Arg Ala
       130                 135                 140
   Ser Arg Arg Arg Gly Ala Arg Pro Ala Phe Ala Pro Val Asp Leu Gly
   145                 150                 155                 160
   Arg Gly Phe Ala Phe Ala Glu Val Glu Leu Tyr Gly Asp Val Val Leu
                   165                 170                 175
   Arg Phe Val Ser His Pro Asp Gly Thr Asp Val Pro Phe Leu Pro Gly
               180                 185                 190
   Phe Glu Gly Val Thr Asn Pro Asp Ala Val Asp Tyr Gly Leu Thr Arg
           195                 200                 205
   Phe Asp His Val Val Gly Asn Val Pro Glu Leu Ala Pro Ala Ala Ala
       210                 215                 220
   Tyr Ile Ala Gly Phe Thr Gly Phe His Glu Phe Ala Glu Phe Thr Ala
   225                 230                 235                 240
   Glu Asp Val Gly Thr Thr Glu Ser Gly Leu Asn Ser Val Val Leu Ala
                   245                 250                 255
   Asn Asn Ser Glu Gly Val Leu Leu Pro Leu Asn Glu Pro Val His Gly
               260                 265                 270
   Thr Lys Arg Arg Ser Gln Ile Gln Thr Phe Leu Glu His His Gly Gly
           275                 280                 285
   Pro Gly Val Gln His Ile Ala Val Ala Ser Ser Asp Val Leu Arg Thr
       290                 295                 300
   Leu Arg Lys Met Arg Ala Arg Ser Ala Met Gly Gly Phe Asp Phe Leu
   305                 310                 315                 320
   Pro Pro Pro Leu Pro Lys Tyr Tyr Glu Gly Val Arg Arg Leu Ala Gly
                   325                 330                 335
   Asp Val Leu Ser Glu Ala Gln Ile Lys Glu Cys Gln Glu Leu Gly Val
               340                 345                 350
   Leu Val Asp Arg Asp Asp Gln Gly Val Leu Leu Gln Ile Phe Thr Lys
           355                 360                 365
   Pro Val Gly Asp Arg Pro Thr Leu Phe Leu Glu Met Ile Gln Arg Ile
       370                 375                 380
   Gly Cys Met Glu Lys Asp Glu Arg Gly Glu Glu Tyr Gln Lys Gly Gly
   385                 390                 395                 400
   Cys Gly Gly Phe Gly Lys Gly Asn Phe Ser Glu Leu Phe Lys Ser Ile
                   405                 410                 415
   Glu Asp Tyr Glu Lys Ser Leu Glu Ala Lys Gln Ser Ala Ala Val Gln
               420                 425                 430
   Gly Ser
<210> 13
   <211> 1332
   <212> DNA
   <213> Zea mays
<220>
   <221> CDS
   <222> (1)..(1332)
<210> 14
   <211> 444
   <212> PRT
   <213> Zea mays
<400> 14
   Met Pro Pro Thr Pro Thr Ala Ala Ala Ala Gly Ala Ala Val Ala Ala
   1               5                   10                  15
   Ala Ser Ala Ala Glu Gln Ala Ala Phe Arg Leu Val Gly His Arg Asn
   20 25 30
   Phe Val Arg Phe Asn Pro Arg Ser Asp Arg Phe His Thr Leu Ala Phe
   35 40 45
   His His Val Glu Leu Trp Cys Ala Asp Ala Ala Ser Ala Ala Gly Arg
   50 55 60
   Phe Ser Phe Gly Leu Gly Ala Pro Leu Ala Ala Arg Ser Asp Leu Ser
   65 70 75 80
   Thr Gly Asn Ser Ala His Ala Ser Leu Leu Leu Arg Ser Gly Ser Leu
   85 90 95
   Ser Phe Leu Phe Thr Ala Pro Tyr Ala His Gly Ala Asp Ala Ala Thr
   100 105 110
   Ala Ala Leu Pro Ser Phe Ser Ala Ala Ala Ala Arg Arg Phe Ala Ala
   115 120 125
   Asp His Gly Leu Ala Val Arg Ala Val Ala Leu Arg Val Ala Asp Ala
   130 135 140
   Glu Asp Ala Phe Arg Ala Ser Val Ala Ala Gly Ala Arg Pro Ala Phe
   145 150 155 160
   Gly Pro Val Asp Leu Gly Arg Gly Phe Arg Leu Ala Glu Val Glu Leu
   165 170 175
   Tyr Gly Asp Val Val Leu Arg Tyr Val Ser Tyr Pro Asp Gly Ala Ala
   180 185 190
   Gly Glu Pro Phe Leu Pro Gly Phe Glu Gly Val Ala Ser Pro Gly Ala
   195 200 205
   Ala Asp Tyr Gly Leu Ser Arg Phe Asp His Ile Val Gly Asn Val Pro
   210 215 220
   Glu Leu Ala Pro Ala Ala Ala Tyr Phe Ala Gly Phe Thr Gly Phe His
   225 230 235 240
   Glu Phe Ala Glu Phe Thr Thr Glu Asp Val Gly Thr Ala Glu Ser Gly
   245 250 255
   Leu Asn Ser Met Val Leu Ala Asn Asn Ser Glu Asn Val Leu Leu Pro
   260 265 270
   Leu Asn Glu Pro Val His Gly Thr Lys Arg Arg Ser Gln Ile Gln Thr
           275                 280                 285
   Phe Leu Asp His His Gly Gly Pro Gly Val Gln His Met Ala Leu Ala
       290                 295                 300
   Ser Asp Asp Val Leu Arg Thr Leu Arg Glu Met Gln Ala Arg Ser Ala
   305                 310                 315                 320
   Met Gly Gly Phe Glu Phe Met Ala Pro Pro Thr Ser Asp Tyr Tyr Asp
                   325                 330                 335
   Gly Val Arg Arg Arg Ala Gly Asp Val Leu Thr Glu Ala Gln Ile Lys
               340                 345                 350
   Glu Cys Gln Glu Leu Gly Val Leu Val Asp Arg Asp Asp Gln Gly Val
           355                 360                 365
   Leu Leu Gln Ile Phe Thr Lys Pro Val Gly Asp Arg Pro Thr Leu Phe
       370                 375                 380
   Leu Glu Ile Ile Gln Arg Ile Gly Cys Met Glu Lys Asp Glu Lys Gly
   385                 390                 395                 400
   Gln Glu Tyr Gln Lys Gly Gly Cys Gly Gly Phe Gly Lys Gly Asn Phe
                   405                 410                 415
   Ser Gln Leu Phe Lys Ser Ile Glu Asp Tyr Glu Lys Ser Leu Glu Ala
               420                 425                 430
   Lys Gln Ala Ala Ala Ala Ala Ala Ala Gln Gly Ser
           435                 440
<210> 15
   <211> 1329
   <212> DNA
   <213> Daucus carota
<220>
   <221> CDS
   <222> (1)..(1329)
<400> 15
   atg ggg aaa aaa caa tcg gaa gct gaa att ctc tca agc aat tca tca        48
   Met Gly Lys Lys Gln Ser Glu Ala Glu Ile Leu Ser Ser Asn Ser Ser
   1               5                   10                  15
   aac acc tct cct gca aca ttc aag ctg gtc ggt ttc aac aac ttc gtc        96
   Asn Thr Ser Pro Ala Thr Phe Lys Leu Val Gly Phe Asn Asn Phe Val
               20                  25                  30
   cgc gcc aac ccc aag tcc gat cac ttc gcc gtg aag cgg ttc cac cac      144
   Arg Ala Asn Pro Lys Ser Asp His Phe Ala Val Lys Arg Phe His His
           35                  40                  45
   att gag ttc tgg tgc ggc gac gcc acc aac acg tcg cgg cgg ttc tcg      192
   Ile Glu Phe Trp Cys Gly Asp Ala Thr Asn Thr Ser Arg Arg Phe Ser
       50                  55                  60
   tgg ggc ctc ggc atg cct ttg gtg gcg aaa tcg gat ctc tct act gga      240
   Trp Gly Leu Gly Met Pro Leu Val Ala Lys Ser Asp Leu Ser Thr Gly
   65                  70                  75                  80
   aac tct gtt cac gct tct tat ctt gtt cgc tcg gcg aat ctc agt ttc      288
   Asn Ser Val His Ala Ser Tyr Leu Val Arg Ser Ala Asn Leu Ser Phe
                   85                  90                  95
   gtc ttc acc gct cct tac tct ccg tcc acg acc act tcc tct ggt tca      336
   Val Phe Thr Ala Pro Tyr Ser Pro Ser Thr Thr Thr Ser Ser Gly Ser
               100                 105                 110
   gct gcc atc ccg tct ttt tcg gca tcg ggt ttt cac tct ttt gcg gcc      384
   Ala Ala Ile Pro Ser Phe Ser Ala Ser Gly Phe His Ser Phe Ala Ala
           115                 120                 125
   aaa cac ggc ctt gct gtt cgg gct att gct ctt gaa gtt gct gac gtg      432
   Lys His Gly Leu Ala Val Arg Ala Ile Ala Leu Glu Val Ala Asp Val
       130                 135                 140
   gct gct gcg ttt gag gcc agt gtt gcg cgt ggg gcc agg ccg gct tcg      480
   Ala Ala Ala Phe Glu Ala Ser Val Ala Arg Gly Ala Arg Pro Ala Ser
   145                 150                 155                 160
   gct cct gtt gaa ttg gac gac cag gcg tgg ttg gct gag gtg gag ttg      528
   Ala Pro Val Glu Leu Asp Asp Gln Ala Trp Leu Ala Glu Val Glu Leu
                   165                 170                 175
   tac gga gat gtg gtc ttg agg ttt gtt agt ttt ggg agg gag gag ggt      576
   Tyr Gly Asp Val Val Leu Arg Phe Val Ser Phe Gly Arg Glu Glu Gly
               180                 185                 190
   ttg ttt ttg cct gga ttc gag gcg gtg gag ggg acg gcg tcg ttt ccg      624
   Leu Phe Leu Pro Gly Phe Glu Ala Val Glu Gly Thr Ala Ser Phe Pro
           195                 200                 205
   gat ttg gat tat gga att aga aga ctt gat cat gcg gtg ggg aat gtt      672
   Asp Leu Asp Tyr Gly Ile Arg Arg Leu Asp His Ala Val Gly Asn Val
       210                 215                 220
   acc gag ttg ggg cct gtg gtg gag tat att aaa ggg ttt acg ggg ttt      720
   Thr Glu Leu Gly Pro Val Val Glu Tyr Ile Lys Gly Phe Thr Gly Phe
   225                 230                 235                 240
   cat gaa ttt gcg gag ttt aca gcg gag gat gtg ggg act ttg gag agt      768
   His Glu Phe Ala Glu Phe Thr Ala Glu Asp Val Gly Thr Leu Glu Ser
                   245                 250                 255
   ggg ttg aat tcg gtg gtg ttg gcg aat aat gag gag atg gtt ctg ttg      816
   Gly Leu Asn Ser Val Val Leu Ala Asn Asn Glu Glu Met Val Leu Leu
               260                 265                 270
   ccc ttg aat gag cct gtg tat ggg acc aag agg aag agt cag ata cag      864
   Pro Leu Asn Glu Pro Val Tyr Gly Thr Lys Arg Lys Ser Gln Ile Gln
           275                 280                 285
   act tac ttg gag cac aat gaa ggg gct gga gtg cag cat ttg gct tta       912
   Thr Tyr Leu Glu His Asn Glu Gly Ala Gly Val Gln His Leu Ala Leu
       290                 295                 300
   gtg agt gag gat att ttt agg act tta agg gag atg agg aag agg agt       960
   Val Ser Glu Asp Ile Phe Arg Thr Leu Arg Glu Met Arg Lys Arg Ser
   305                 310                 315                 320
   tgc ctt ggt ggt ttt gag ttt atg cct tcg cca ccg cct acg tat tac      1008
   Cys Leu Gly Gly Phe Glu Phe Met Pro Ser Pro Pro Pro Thr Tyr Tyr
                   325                 330                 335
   aag aat ttg aag aat agg gtc ggg gat gtg ttg agt gat gaa cag atc      1056
   Lys Asn Leu Lys Asn Arg Val Gly Asp Val Leu Ser Asp Glu Gln Ile
               340                 345                 350
   aag gag tgt gaa gat ttg ggg att ttg gtg gat agg gat gat cag ggt      1104
   Lys Glu Cys Glu Asp Leu Gly Ile Leu Val Asp Arg Asp Asp Gln Gly
           355                 360                 365
   aca ttg ctt caa atc ttt acc aag cct gta ggt gac agg cct acc tta      1152
   Thr Leu Leu Gln Ile Phe Thr Lys Pro Val Gly Asp Arg Pro Thr Leu
       370                 375                 380
   ttc ata gag atc att cag agg gta ggg tgc atg ctc aag gac gat gca      1200
   Phe Ile Glu Ile Ile Gln Arg Val Gly Cys Met Leu Lys Asp Asp Ala
   385                 390                 395                 400
   ggg cag atg tac cag aag ggc ggg tgc gga gga ttt ggg aag ggg aac      1248
   Gly Gln Met Tyr Gln Lys Gly Gly Cys Gly Gly Phe Gly Lys Gly Asn
                   405                 410                 415
   ttc tca gag ctg ttc aag tcc atc gaa gaa tat gaa aaa aca ctt gaa      1296
   Phe Ser Glu Leu Phe Lys Ser Ile Glu Glu Tyr Glu Lys Thr Leu Glu
               420                 425                 430
   gct aaa caa atc act gga tct gct gct gca tga                          1329
   Ala Lys Gln Ile Thr Gly Ser Ala Ala Ala
           435                 440
<210> 16
   <211> 442
   <212> PRT
   <213> Daucus carota
<400> 16
   Met Gly Lys Lys Gln Ser Glu Ala Glu Ile Leu Ser Ser Asn Ser Ser
   1               5                   10                  15
   Asn Thr Ser Pro Ala Thr Phe Lys Leu Val Gly Phe Asn Asn Phe Val
               20                  25                  30
   Arg Ala Asn Pro Lys Ser Asp His Phe Ala Val Lys Arg Phe His His
           35                  40                  45
   Ile Glu Phe Trp Cys Gly Asp Ala Thr Asn Thr Ser Arg Arg Phe Ser
       50                  55                  60
   Trp Gly Leu Gly Met Pro Leu Val Ala Lys Ser Asp Leu Ser Thr Gly
   65                  70                  75                  80
   Asn Ser Val His Ala Ser Tyr Leu Val Arg Ser Ala Asn Leu Ser Phe
                   85                  90                  95
   Val Phe Thr Ala Pro Tyr Ser Pro Ser Thr Thr Thr Ser Ser Gly Ser
               100                 105                 110
   Ala Ala Ile Pro Ser Phe Ser Ala Ser Gly Phe His Ser Phe Ala Ala
           115                 120                 125
   Lys His Gly Leu Ala Val Arg Ala Ile Ala Leu Glu Val Ala Asp Val
       130                 135                 140
   Ala Ala Ala Phe Glu Ala Ser Val Ala Arg Gly Ala Arg Pro Ala Ser
   145                 150                 155                 160
   Ala Pro Val Glu Leu Asp Asp Gln Ala Trp Leu Ala Glu Val Glu Leu
                   165                 170                 175
   Tyr Gly Asp Val Val Leu Arg Phe Val Ser Phe Gly Arg Glu Glu Gly
               180                 185                 190
   Leu Phe Leu Pro Gly Phe Glu Ala Val Glu Gly Thr Ala Ser Phe Pro
           195                 200                 205
   Asp Leu Asp Tyr Gly Ile Arg Arg Leu Asp His Ala Val Gly Asn Val
       210                 215                 220
   Thr Glu Leu Gly Pro Val Val Glu Tyr Ile Lys Gly Phe Thr Gly Phe
   225                 230                 235                 240
   His Glu Phe Ala Glu Phe Thr Ala Glu Asp Val Gly Thr Leu Glu Ser
                   245                 250                 255
   Gly Leu Asn Ser Val Val Leu Ala Asn Asn Glu Glu Met Val Leu Leu
               260                 265                 270
   Pro Leu Asn Glu Pro Val Tyr Gly Thr Lys Arg Lys Ser Gln Ile Gln
           275                 280                 285
   Thr Tyr Leu Glu His Asn Glu Gly Ala Gly Val Gln His Leu Ala Leu
       290                 295                 300
   Val Ser Glu Asp Ile Phe Arg Thr Leu Arg Glu Met Arg Lys Arg Ser
   305 310 315 320
   Cys Leu Gly Gly Phe Glu Phe Met Pro Ser Pro Pro Pro Thr Tyr Tyr
   325 330 335
   Lys Asn Leu Lys Asn Arg Val Gly Asp Val Leu Ser Asp Glu Gln Ile
   340 345 350
   Lys Glu Cys Glu Asp Leu Gly Ile Leu Val Asp Arg Asp Asp Gln Gly
   355 360 365
   Thr Leu Leu Gln Ile Phe Thr Lys Pro Val Gly Asp Arg Pro Thr Leu
   370 375 380
   Phe Ile Glu Ile Ile Gln Arg Val Gly Cys Met Leu Lys Asp Asp Ala
   385 390 395 400
   Gly Gln Met Tyr Gln Lys Gly Gly Cys Gly Gly Phe Gly Lys Gly Asn
   405 410 415
   Phe Ser Glu Leu Phe Lys Ser Ile Glu Glu Tyr Glu Lys Thr Leu Glu
   420 425 430
   Ala Lys Gln Ile Thr Gly Ser Ala Ala Ala
   435 440
<210> 17
   <211> 1146
   <212> DNA
   <213> Streptomyces avermitilis
<220>
   <221> CDS
   <222> (1)..(1146)
<400> 17
   atg acg cag acc aca cac cac act ccc gac acc gcc cgg cag gcc gac       48
   Met Thr Gln Thr Thr His His Thr Pro Asp Thr Ala Arg Gln Ala Asp
   1               5                   10                  15
   ccc ttc ccg gtg aag gga atg gac gcg gtc gtc ttc gcc gta ggc aac       96
   Pro Phe Pro Val Lys Gly Met Asp Ala Val Val Phe Ala Val Gly Asn
               20                  25                  30
   gcc aag cag gcc gcg cac tac tac tcc acc gcc ttc ggc atg cag ctt      144
   Ala Lys Gln Ala Ala His Tyr Tyr Ser Thr Ala Phe Gly Met Gln Leu
           35                  40                  45
   gtg gcg tac tcc gga ccg gag aac ggc agc cgc gag acc gct tcg tac      192
   Val Ala Tyr Ser Gly Pro Glu Asn Gly Ser Arg Glu Thr Ala Ser Tyr
       50                  55                  60
   gtc ctc acc aac ggc tcg gca cgc ttc gtc ctc acc tcc gtc atc aag      240
   Val Leu Thr Asn Gly Ser Ala Arg Phe Val Leu Thr Ser Val Ile Lys
   65                  70                  75                  80
   ccc gcc acc ccc tgg ggc cac ttc ctc gcc gac cat gtg gcc gag cac      288
   Pro Ala Thr Pro Trp Gly His Phe Leu Ala Asp His Val Ala Glu His
                   85                  90                  95
   ggc gac ggc gtc gtc gac ctc gcc atc gag gtc ccg gac gcc cgc gcc      336
   Gly Asp Gly Val Val Asp Leu Ala Ile Glu Val Pro Asp Ala Arg Ala
               100                 105                 110
   gcc cac gcg tac gcg atc gag cac ggc gcc cgc tcg gtc gcc gag ccg      384
   Ala His Ala Tyr Ala Ile Glu His Gly Ala Arg Ser Val Ala Glu Pro
           115                 120                 125
   tac gag ctg aag gac gag cac ggc acg gtc gtc ctc gcc gcg atc gcc      432
   Tyr Glu Leu Lys Asp Glu His Gly Thr Val Val Leu Ala Ala Ile Ala
       130                 135                 140
   acc tac ggc aag acc cgc cac acc ctc gtc gac cgg acc ggc tac gac      480
   Thr Tyr Gly Lys Thr Arg His Thr Leu Val Asp Arg Thr Gly Tyr Asp
   145                 150                 155                 160
   ggc ccc tac ctc ccc ggc tac gtg gcc gcc gcc ccg atc gtc gaa ccg      528
   Gly Pro Tyr Leu Pro Gly Tyr Val Ala Ala Ala Pro Ile Val Glu Pro
                   165                 170                 175
   ccc gcc cac cgc acc ttc cag gcc atc gac cac tgc gtc ggc aac gtc      576
   Pro Ala His Arg Thr Phe Gln Ala Ile Asp His Cys Val Gly Asn Val
               180                 185                 190
   gag ctc ggc cgg atg aac gaa tgg gtc ggc ttc tac aac aag gtc atg      624
   Glu Leu Gly Arg Met Asn Glu Trp Val Gly Phe Tyr Asn Lys Val Met
           195                 200                 205
   ggc ttc acg aac atg aag gag ttc gtg ggc gac gac atc gcg acc gag      672
   Gly Phe Thr Asn Met Lys Glu Phe Val Gly Asp Asp Ile Ala Thr Glu
       210                 215                 220
   tac tcg gcg ctg atg tcg aag gtc gtg gcc gac ggc acg ctc aag gtc      720
   Tyr Ser Ala Leu Met Ser Lys Val Val Ala Asp Gly Thr Leu Lys Val
   225                 230                 235                 240
   aag ttc ccg atc aac gag ccc gcc ctc gcc aag aag aag tcc cag atc      768
   Lys Phe Pro Ile Asn Glu Pro Ala Leu Ala Lys Lys Lys Ser Gln Ile
                   245                 250                 255
   gac gag tac ctg gag ttc tac ggc ggc gcg ggc gtc cag cac atc gcg      816
   Asp Glu Tyr Leu Glu Phe Tyr Gly Gly Ala Gly Val Gln His Ile Ala
               260                 265                 270
   ctg aac acg ggt gac atc gtc gag acg gta cgc acg atg cgc gcc gcc      864
   Leu Asn Thr Gly Asp Ile Val Glu Thr Val Arg Thr Met Arg Ala Ala
           275                 280                 285
   ggc gtc cag ttc ctg gac acg ccc gac tcg tac tac gac acc ctc ggg      912
   Gly Val Gln Phe Leu Asp Thr Pro Asp Ser Tyr Tyr Asp Thr Leu Gly
       290                 295                 300
   gag tgg gtg ggc gac acc cgc gtc ccc gtc gac acc ctg cgc gag ctg      960
   Glu Trp Val Gly Asp Thr Arg Val Pro Val Asp Thr Leu Arg Glu Leu
   305                 310                 315                 320
   aag atc ctc gcg gac cgc gac gag gac ggc tat ctg ctc cag atc ttc      1008
   Lys Ile Leu Ala Asp Arg Asp Glu Asp Gly Tyr Leu Leu Gln Ile Phe
                   325                 330                 335
   acc aag ccg gtc cag gac cgc ccg acg gtc ttc ttc gag atc atc gaa      1056
   Thr Lys Pro Val Gln Asp Arg Pro Thr Val Phe Phe Glu Ile Ile Glu
               340                 345                 350
   cgc cac ggc tcg atg gga ttc ggc aag ggc aac ttc aag gcc ctg ttc      1104
   Arg His Gly Ser Met Gly Phe Gly Lys Gly Asn Phe Lys Ala Leu Phe
           355                 360                 365
   gag gcg atc gag cgg gag cag gag aag cgg ggc aac ctg tag              1146
   Glu Ala Ile Glu Arg Glu Gln Glu Lys Arg Gly Asn Leu
       370                 375                 380
<210> 18
   <211> 381
   <212> PRT
   <213> Streptomyces avermitilis
<400> 18
   Met Thr Gln Thr Thr His His Thr Pro Asp Thr Ala Arg Gln Ala Asp
   1               5                   10                  15
   Pro Phe Pro Val Lys Gly Met Asp Ala Val Val Phe Ala Val Gly Asn
               20                  25                  30
   Ala Lys Gln Ala Ala His Tyr Tyr Ser Thr Ala Phe Gly Met Gln Leu
           35                  40                  45
   Val Ala Tyr Ser Gly Pro Glu Asn Gly Ser Arg Glu Thr Ala Ser Tyr
       50                  55                  60
   Val Leu Thr Asn Gly Ser Ala Arg Phe Val Leu Thr Ser Val Ile Lys
   65                  70                  75                  80
   Pro Ala Thr Pro Trp Gly His Phe Leu Ala Asp His Val Ala Glu His
                   85                  90                  95
   Gly Asp Gly Val Val Asp Leu Ala Ile Glu Val Pro Asp Ala Arg Ala
               100                 105                 110
   Ala His Ala Tyr Ala Ile Glu His Gly Ala Arg Ser Val Ala Glu Pro
           115                 120                 125
   Tyr Glu Leu Lys Asp Glu His Gly Thr Val Val Leu Ala Ala Ile Ala
       130                 135                 140
   Thr Tyr Gly Lys Thr Arg His Thr Leu Val Asp Arg Thr Gly Tyr Asp
   145                 150                 155                 160
   Gly Pro Tyr Leu Pro Gly Tyr Val Ala Ala Ala Pro Ile Val Glu Pro
                   165                 170                 175
   Pro Ala His Arg Thr Phe Gln Ala Ile Asp His Cys Val Gly Asn Val
               180                 185                 190
   Glu Leu Gly Arg Met Asn Glu Trp Val Gly Phe Tyr Asn Lys Val Met
           195                 200                 205
   Gly Phe Thr Asn Met Lys Glu Phe Val Gly Asp Asp Ile Ala Thr Glu
       210                 215                 220
   Tyr Ser Ala Leu Met Ser Lys Val Val Ala Asp Gly Thr Leu Lys Val
   225                 230                 235                 240
   Lys Phe Pro Ile Asn Glu Pro Ala Leu Ala Lys Lys Lys Ser Gln Ile
                   245                 250                 255
   Asp Glu Tyr Leu Glu Phe Tyr Gly Gly Ala Gly Val Gln His Ile Ala
               260                 265                 270
   Leu Asn Thr Gly Asp Ile Val Glu Thr Val Arg Thr Met Arg Ala Ala
           275                 280                 285
   Gly Val Gln Phe Leu Asp Thr Pro Asp Ser Tyr Tyr Asp Thr Leu Gly
       290                 295                 300
   Glu Trp Val Gly Asp Thr Arg Val Pro Val Asp Thr Leu Arg Glu Leu
   305                 310                 315                 320
   Lys Ile Leu Ala Asp Arg Asp Glu Asp Gly Tyr Leu Leu Gln Ile Phe
                   325                 330                 335
   Thr Lys Pro Val Gln Asp Arg Pro Thr Val Phe Phe Glu Ile Ile Glu
               340                 345                 350
   Arg His Gly Ser Met Gly Phe Gly Lys Gly Asn Phe Lys Ala Leu Phe
           355                 360                 365
   Glu Ala Ile Glu Arg Glu Gln Glu Lys Arg Gly Asn Leu
       370                 375                 380
<210> 19
   <211> 45
   <212> DNA
   <213> Artificial
<220>
   <223> primer sequence
<400> 19
   ccatggctca tcaccatcac catcaccaaa acgccgccgt ttcag 45
<210> 20
   <211> 27
   <212> DNA
   <213> artificial
<220>
   <223> primer sequence
<400> 20
   tctagatcat cccactaact gtttggc 27
<210> 21
   <211> 51
   <212> DNA
   <213> artificial
<220>
   <223> primer sequence
<400> 21
   ccatggctca tcaccatcac catcacgcag atctatacga aaacccaatg g 51
<210> 22
   <211> 29
   <212> DNA
   <213> artificial
<220>
   <223> primer sequence
<400> 22
   tctagattaa tcggcggtca atacaccac 29
<210> 23
   <211> 33
   <212> DNA
   <213> artificial
<220>
   <223> primer sequence
<220>
   <221> misc_feature
   <222> (16)..(18)
   <223> n is a, c, g, or t
<400> 23
   ggtggttttg gcaaannnaa tttctctgag ctc 33
<210> 24
   <211> 33
   <212> DNA
   <213> artificial
<220>
   <223> primer sequence
<220>
   <221> misc_feature
   <222> (16)..(18)
   <223> n is a, c, g, or t
<400> 24
   gagctcagag aaattnnntt tgccaaaacc acc 33
<210> 25
   <211> 33
   <212> DNA
   <213> artificial
<220>
   <223> primer sequence
<220>
   <221> misc_feature
   <222> (16)..(18)
   <223> n is a, c, g, or t
<400> 25
   cagcgccttg aagttnnnct cgccaaaccc atc 33
<210> 26
   <211> 33
   <212> DNA
   <213> artificial
<220>
   <223> primer sequence
<220>
   <221> misc_feature
   <222> (16)..(18)
   <223> n is a, c, g, or t
<400> 26
   gatgggtttg gcgagnnnaa cttcaaggcg ctg 33
<210> 27
   <211> 22
   <212> DNA
   <213> artificial
<220>
   <223> primer sequence
<400> 27
   gatcttctcg gaaaccctga tg 22
<210> 28
   <211> 22
   <212> DNA
   <213> artificial
<220>
   <223> primer sequence
<400> 28
   gggattcttg tagacagaga tg 22
<210> 29
   <211> 20
   <212> DNA
   <213> artificial
<220>
   <223> primer sequence
<400> 29
   cccactaact gtttggcttc 20
<210> 30
   <211> 20
   <212> DNA
   <213> artificial
<220>
   <223> primer sequence
<400> 30
   ggcggtcaat acaccacgac 20
<210> 31
   <211> 23
   <212> DNA
   <213> artificial
<220>
   <223> primer sequence
<400> 31
   gactcgaaca gcgccttgaa gtt 23
<210> 32
   <211> 18
   <212> DNA
   <213> artificial
<220>
   <223> primer sequence
<400> 32
   ggatgtggtg gttttggc 18

## Description

The present invention relates to a nucleic acid sequence encoding a mutated hydroxyphenylpyruvate dioxygenase (HPPD), to a chimeric gene which comprises this sequence as the coding sequence, and to its use for obtaining plants which are resistant to HPPD inhibitor herbicides.

The hydroxyphenylpyruvate dioxygenases (HPPD; EC 1.13.11.27) are enzymes which catalyse the reaction in which para-hydroxyphenylpyruvate (HPP), a tyrosine degradation product, is transformed into homogentisate (HG), the precursor in plants of tocopherol and plastoquinone (Crouch N.P. et al., 1997; Fritze et al., 2004). Tocopherol acts as a membrane-associated antioxidant. Plastoquinone, firstly acts as an electron carrier between PSII and the cytochrome b6/f complex and secondly, is a redox cofactor for phytoene desaturase, which is involved in the biosynthesis of carotenoids.
Most plants synthesize tyrosine via arrogenate (Abou-Zeid *et al*. 1995; Bonner *et al*., 1995; Byng *et al*., 1981; Connely and Conn 1986; Gaines *et al*., 1982). In these plants, the HPP is derived only from the degradation of tyrosine. On the other hand, in organisms such as the yeast *Sacharomyces cerevisiae* or the bacterium *Escherichia coli*, HPP is a tyrosine precursor, and it is synthesized by the action of an enzyme, prephenate dehydrogenase (hereinafter referred to as PDH), which converts prephenate to HPP (Lingens *et al*., 1967; Sampathkumar and Morrisson 1982). In these organisms, the production of HPP is therefore directly connected to the aromatic amino acid biosynthetic pathway (shikimate pathway), and not to the tyrosine degradation pathway.

Inhibition of HPPD leads to uncoupling of photosynthesis, deficiency in accessory light-harvesting pigments and, most importantly, to destruction of chlorophyll by UV-radiation and reactive oxygen species due to the lack of photo protection normally provided by carotenoids (Norris et al. 1995). Photo bleaching of photosynthetically active tissues leads to growth inhibition and plant death.

Some molecules which inhibit HPPD, and which bind specifically to the enzyme in order to inhibit transformation of the HPP into homogentisate, have proven to be very effective selective herbicides.

Most commercially available HPPD inhibitor herbicides belong to one of these four chemical families:
1) the triketones, e.g. sulcotrione [i.e. 2-[2-chloro-4-(methylsulfonyl)benzoyl]-1,3-cyclohexanedione], mesotrione [i.e.2-[4-(methylsulfonyl)-2-nitrobenzoyl]-1,3-cyclohexanedione], tembotrione [i.e.2-[2-chloro-4-(methylsulfonyl)-3-[(2,2,2,-tri-fluoroethoxy)methyl] benzoyl]-1,3-cyclo-hexanedione];
2) The diketonitriles, e.g. 2-cyano-3-cyclopropyl-1-(2-methylsulphonyl-4-trifluoromethylphenyl)-propane-1,3-dione and 2-cyano-1-[4-(methylsulphonyl)-2-trifluoromethylphenyl]-3-(1-methylcyclopropyl)propane-1,3-fione;
2) the isoxazoles, e.g. isoxaflutole [i.e.(5-cyclopropyl-4-isoxazolyl)[2-(methylsulfonyl)-4-(trifluoromethyl)phenyl]methanone]. In plants, the isoxaflutole is rapidly metabolized in DKN, a diketonitrile compound which exhibits the HPPD inhibitor property; and
4) the pyrazolinates, e.g. topramezone [i.e. [3-(4,5-dihydro-3-isoxazolyl)-2-methyl-4-(methylsulfonyl) phenyl] (5-hydroxy-1-methyl-1H-pyrazol-4-yl)methanone], and pyrasulfotole [(5-hydroxy-1,3-dimethylpyrazol-4-yl(2-mesyl-4-trifluaromethylphenyl)methanone].

These HPPD-inhibiting herbicides can be used against grass and / or broad leaf weeds in crop plants that display metabolic tolerance, such as maize (Zea mays) in which they are rapidly degraded (Schulz et al., 1993; Mitchell et al., 2001; Garcia et al., 2000; Pallett et al., 2001). In order to extend the scope of these HPPD-inhibiting herbicides, several efforts have been developed in order to confer to plants, particularly plants without or with an under performing metabolic tolerance, an agricultural level tolerance to them.

Besides the attempt of by-passing HPPD-mediated production of homogentisate (US 6,812,010), overexpressing the sensitive enzyme so as to produce quantities of the target enzyme in the plant which are sufficient in relation to the herbicide has been performed (WO96/38567). Overexpression of HPPD resulted in better pre-emergence tolerance to the diketonitrile derivative (DKN) of Isoxaflutole (IFT), but tolerance was not sufficient for tolerance to post-emergence treatment (Matringe et al., 2005).

A third strategy was to mutate the HPPD in order to obtain a target enzyme which, while retaining its properties of catalysing the transformation of HPP into homogentisate, is less sensitive to HPPD inhibitors than is the native HPPD before mutation. This strategy has been successfully applied for the production of plants tolerant to 2-cyano-3-cyclopropyl-1-(2-methylsulphonyl-4-trifluoromethylphenyl)-propane-1,3-dione and to 2-cyano-1-[4-(methylsulphonyl)-2-trifluoromethylphenyl]-3-(1-methylcyclopropyl)propane-1,3-fione (EP496630), two HPPD-inhibiting herbicides belonging to the diketonitriles family (WO 99/24585). Pro215Leu, Gly336Glu, Gly336Ile, and more particularly Gly336Trp (positions of the mutated amino acid are indicated with reference to the *Pseudomonas* HPPD of SEQ ID NO:2) were identified as mutations which are responsible for an increased tolerance to pre-emergence treatment with these diketonitrile herbicides without causing an alteration of the activity of the enzyme.

More recently, introduction of a *Pseudomonas* HPPD gene into the plastid genome of tobacco and soybean has shown to be much more effective than nuclear transformation, conferring even tolerance to post-emergence application of isoxaflutol (Dufourmantel et al., 2007).

In WO 04/024928, the inventors have sought to increase the prenylquinone biosynthesis (e.g., synthesis of plastoquinones, tocopherols) in the cells of plants by increasing the flux of the HPP precursor into the cells of these plants. This has been done by connecting the synthesis of said precursor to the "shikimate" pathway by overexpression of a PDH enzyme. They have also noted that the transformation of plants with a gene encoding a PDH enzyme makes it possible to increase the tolerance of said plants to HPPD inhibitors.

Despite these successes obtained for the development of plants showing tolerance to diketonitrile herbicides, it is still necessary to develop and/or improve the system of tolerance to HPPD inhibitors, particularly for HPPD inhibitors belonging to the classes of the triketones (e.g.sulcotrione, mesotrione, and tembotrione) and the pyrazolinates (e.g.topramezone and pyrasulfotole).

The present invention therefore relates to novel mutated HPPD enzymes which retain their properties of catalysing the conversion of para-hydroxyphenylpyruvate (HPP) to homogentisate and which are less sensitive to tembotrione than the original unmutated HPPD, characterized in that they contain a mutation at the position 336 (amino acid glycine in the native HPPD) with reference to the *Pseudomonas* HPPD of SEQ ID NO:2 which is Gly336Phe.

Here is also described the HPPD enzyme from a plant which contains a mutation on glycine at position 422 with reference to the amino acid sequence of the *Arabidopsis* HPPD of SEQ ID NO:4 (i.e. position 336 with reference to the amino acid sequence of the Pseudomonas HPPD of SEQ ID NO:2) which is Gly336Phe.

It is noted than the position 336 with reference to the *Pseudomonas* HPPD of SEQ ID NO:2 is the position 422 with reference to the *Arabidospis thaliana* HPPD of SEQ ID NO:4.

In another particular embodiment, the mutated HPPD of the invention contains a second mutation, in addition to the first mutation on the amino acid glycine at the position 336 with reference to the *Pseudomonas* HPPD of SEQ ID NO:2.

In a more particular embodiment, the second mutated amino acid is selected from the selected amino acids: Pro215, Gly298, Gly332, Phe333, Gly334 and Asn337, with reference to the *Pseudomonas* HPPD sequence of SEQ ID NO:2.

Also, the present invention provides mutated HPPD enzymes which retain their properties of catalysing the conversion of para-hydroxyphenylpyruvate (HPP) to homogentisate and which are less sensitive to tembotrione than the original unmutated HPPD, characterized in that they contain a mutation of the amino acid glycine at the position 336 with reference to the *Pseudomonas* HPPD of SEQ ID NO:2, as well as uses of such enzymes to render plants tolerant to HPPD inhibitors, processes wherein triketones or pyrazolinates herbicides are applied to plants expressing such mutant enzymes, and plants tolerant to such HPPD inhibitors of the class of triketones or pyrazolinates by comprising in their genome a gene encoding certain HPPD enzymes mutated in the position 336 with reference to the *Pseudomonas* HPPD of SEQ ID NO.2 which is Gly336Phe.

In a particular embodiment of the invention, the mutated HPPD enzyme is less sensitive to tembotrione than the original unmutated HPPD and is mutated in the position 336 with reference to the *Pseudomonas* HPPD of SEQ ID NO:2 according to a mutation which is Gly336Phe.

Several HPPDs and their primary sequences have been described in the state of the art, in particular the HPPDs of bacteria such as *Pseudomonas* (Rüetschi et al., Eur. J. Biochem., 205, 459-466, 1992, WO 96/38567), of plants such as *Arabidopsis* (WO 96/38567, Genebank AF047834), carrot (WO 96/38567, Genebank 87257), Avena sativa (WO 02/046387), wheat (WO 02/046387), Brachiaria platyphylla (WO 02/046387), Cenchrus echinatus (WO 02/046387), Lolium rigidum (WO 02/046387), Festuca arundinacea (WO 02/046387), Setaria faberi (WO 02/046387), Eleusine indica (WO 02/046387), and Sorghum (WO 02/046387), of *Coccicoides* (Genebank COITRP) or of mammals such as the mouse or the pig.

By aligning these known sequences, by using the customary means of the art, such as, for example, the method described by Thompson, J.D. et al. (CLUSTAL W: improving the sensitivity of progressive multiple sequence alignment through sequence weighting, positions-specific gap penalties and weight matrix choice. Nucleic Acids Research, 22; 4673-4680, 1994), and accessing these computer programs for sequence alignment which are accessible via the Internet, for example, the skilled person is able to define the sequence homologies in relation to a reference sequence and find the key amino acids or else define common regions.

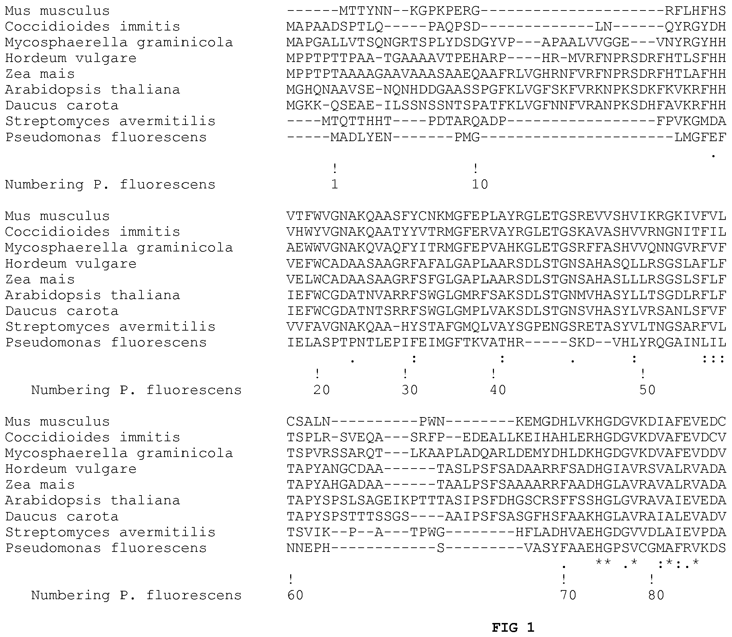

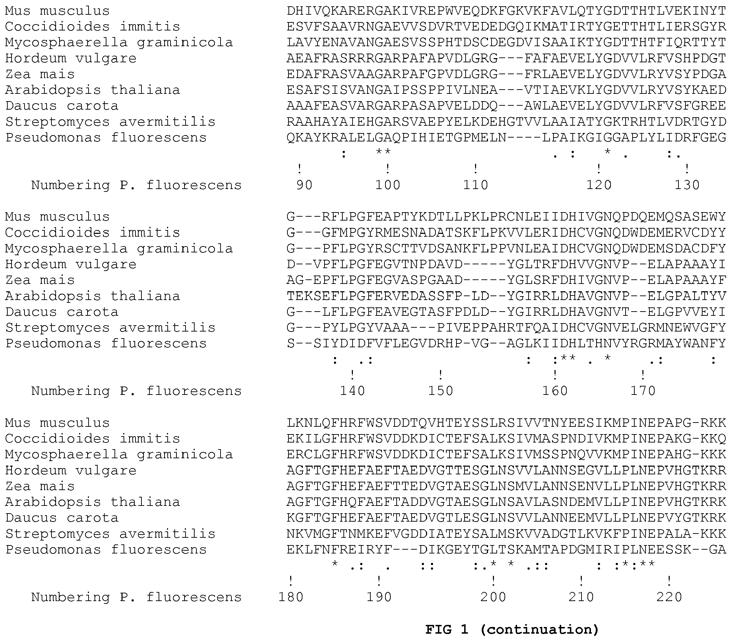

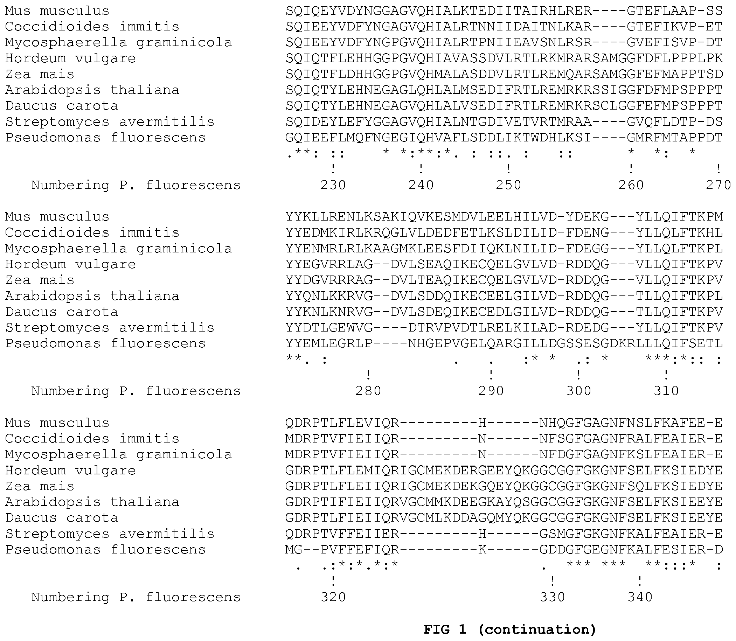

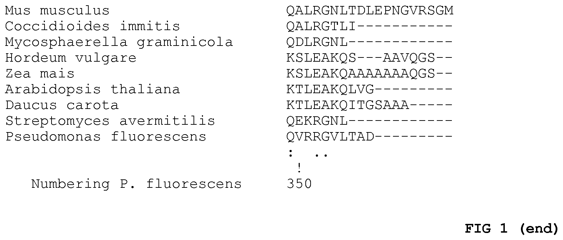

In the case of the present invention, the reference sequence is the *Pseudomonas* sequence, with all the definitions and indications of the positions of particular amino acids being made with respect to the primary *Pseudomonas* HPPD sequence of SEQ ID NO: 2, except when specifically indicated. The attached Figure 1 depicts an alignment of several HPPD sequences which are described in the state of the art; these sequences are aligned with respect to the *Pseudomonas* HPPD sequence as the reference sequence and comprise the HPPD sequences of *Streptomyces avermitilis* (Genebank SAV11864), of *Daucus carota* (Genebank DCU 87257), of *Arabidopsis thaliana* (Genebank AF047834), of *Zea mais*, of *Hordeum vulgare* (Genebank HVAJ693), of *Mycosphaerella graminicola* (Genebank AF038152), of *Coccicoides immitis* (Genebank COITRP) and of *Mus musculus* (Genebank MU54HD) This figure gives the numbering of the amino acids of the *Pseudomonas* sequence and also the amino acids which are common to these sequences, with these amino acids being designated by an asterisk. On the basis of such an alignment, it is easy, from the definition of the *Pseudomonas* amino acid by its position and its nature, to identify the position of the corresponding amino acid in another HPPD sequence. Figure 1 shows that this can be done with the alignment of sequences of different plant, mammalian and bacterial origin, demonstrating that this method of alignment, which is well known to a skilled person, can be generalized to any other sequence. An alignment of different HPPD sequences is also described in Patent Application WO 97/49816.
In WO99/24585, the analysis of the tertiary structure of the *Pseudomonas* HPPD monomer shows the presence of a C-terminal part of the HPPDs, which is where the active site of the enzyme is located, linked to its N-terminal part by a linking peptide which ensures the stability of the enzyme and its oligomerization (the *Pseudomonas* HPPD is a tetramer while the plant HPPDs are dimers). This structure was obtained by the customary methods of studying crystal X-ray diffraction. The linking peptide makes it possible to define the N-terminal end of the C-terminal part of the enzyme, with the said linking peptide being located between amino acids 145 and 157 in the case of *Pseudomonas* (cf. Figure 1). Two amino acids, which are in positions 161 and 162 in the case of the *Pseudomonas* sequence (D = Asp161 and H = His162), will be noted in all sequences shown in the sequence alignment depicted in the attached Figure 1. With reference to the *Pseudomonas* HPPD, it is therefore possible to define the linking peptide as being located between approximately 5 and 15 amino acids upstream of the amino acid Asp161.

According to the invention, "mutated HPPD" is understood as being the replacement of at least one amino acid of the primary sequence of the HPPD with another amino acid. The expression "mutated amino acid" will be used below to designate the amino acid which is replaced by another amino acid, thereby designating the site of the mutation in the primary sequence of the protein.
According to the invention, the mutation is effected on the amino acid glycine at position 336 with reference to the *Pseudomonas* sequence of SEQ ID NO: 2, which is common to almost all the identified HPPD sequences. On 240 HPPD sequences known so far, 238 contain a glycine at position 336, and only the HPPD sequences of Synechococcus sp. JA-3-3Ab (Acc-No Q2JX04) and Synechococcus sp. JA-2-3B'a(2-13) (Acc-No Q2JPN8)) have an alanine at this postion. Gly336 is part of a consensus sequence "Gly-Phe-Gly-X-Gly-Asn-Phe" found in most of the HPPD sequences, wherein X can be any of the 20 amino acids, among the HPPDs from various origins, which makes the identification of the Gly336 feasible without any difficulties in HPPDs from any source by the sequence alignment method.

As an example, Gly336 with reference to the *Pseudomonas* sequence is Gly422 with reference to the *Arabidospsis thaliana* sequence of SEQ ID NO: 4 (see figure 1), but herein reference will be made to Gly at reference position 336 by reference to the Pseudomonas sequence of SEQ ID NO: 2 (except when specifically indicated), even though the mutation can be in any useful HPPD enzyme in accordance with this invention, not necessarily in the Pseudomonas HPPD.

The enzymatic activity of HPPDs can be measured by any method that makes it possible either to measure the decrease in the amount of the HPP or O₂ substrates, or to measure the accumulation of any of the products derived from the enzymatic reaction, i.e. homogentisate or CO₂. In particular, the HPPD activity can be measured by means of the method described in Garcia et al. (1997) or Garcia et al. (1999.

According to the invention, a HPPD inhibitor of the class of triketones (or triketone HPPD inhibitor) means a HPPD inhibitor having a triketone skeleton. As an example of such triketone HPPD inhibitor, one can cite the molecules sulcotrione [i.e. 2-[2-chloro-4-(methylsulfonyl)benzoyl]-1,3-cyclohexanedione], mesotrione [i.e.2-[4-(methylsulfonyl)-2-nitrobenzoyl]-1,3-cyclohexanedione], and tembotrione [i.e.2-[2-chloro-4-(methylsulfonyl)-3-[(2,2,2,-tri-fluoroethoxy)methyl]benzoyl]-1,3-cyclo-hexanedione].

A HPPD of the class of pyrazolinates(or pyrazolinate HPPD inhibitor) means a HPPD inhibitor having a pyrazole radical. As an example of such pyrazolinates HPPD inhibitor, one can cite the molecules topramezone [i.e.[3-(4,5-dihydro-3-isoxazolyl)-2-methyl-4-(methylsulfonyl)phenyl](5-hydroxy-1-methyl-1H-pyrazol-4-yl)methanone] and pyrasulfotole [(5-hydroxy-1,3-dimethylpyrazol-4-yl(2-mesyl-4-trifluaromethylphenyl)methanone].
In a further embodiment of the invention, HPPD is mutated at a second amino acid position in addition to the mutation of Gly336. The presence of this second mutation may further increase the tolerance to the same HPPD inhibitor herbicide than the one for which the first mutation is conferring a tolerance, or may confer tolerance to a second HPPD inhibitor herbicide. Examples of such mutations conferring tolerance to HPPD inhibitors, and in particular to diketonitriles and to the isoxaflutole, are described in WO 99/24585.

In a particular embodiment of the invention, the second mutated amino acid is selected from the following reference amino acids, with reference to the *Pseudomonas* sequence of SEQ ID NO: 2: Pro215, Gly332, Phe333, Gly334 and Asn337, and also Gly298 in the Pseudomonas sequence (this last having no counterpart in other HPPDs, see Fig 1).

In one embodiment of the invention, the second mutated amino acid is Pro215 with reference to the *Pseudomonas* sequence of SEQ ID NO: 2, and the mutation is particularly Pro215Leu.

The present invention also relates to a nucleic acid sequence, particularly an isolated DNA, which encodes a mutated HPPD as described above.

The present invention also relates to a nucleic acid sequence encoding a mutated HPPD enzyme which retains their properties of catalysing the conversion of para-hydroxyphenylpyruvate (HPP) to homogentisate and which is less sensitive to tembotrione than the original unmutated HPPD, characterized in that it contains a mutation of the amino acid glycine at the position 336 with reference to the *Pseudomonas* HPPD of SEQ ID NO:2 which is Gly336Phe.

In a more particular embodiment, the nucleic acid sequence of the invention encodes a mutated HPPD enzyme which is less sensitive to tembotrione than the original unmutated HPPD and wherein the HPPD is mutated in the position 336 with reference to the *Pseudomonas* HPPD of SEQ ID NO:2 according to a mutation which is Gly336Phe.

In an even more particular embodiment, the nucleic acid sequence of the invention encodes a mutated HPPD enzyme which is less sensitive to tembotrione than the original unmutated HPPD and wherein the HPPD is mutated in the position 336 with reference to the *Pseudomonas* HPPD of SEQ ID NO:2 according to a mutation which is Gly336Phe.

According to the present invention, a "nucleic acid sequence" is understood as being a nucleotide sequence which can be of the DNA or RNA type, preferably of the DNA type, and in particular double-stranded, whether it be of natural or synthetic origin, in particular a DNA sequence in which the codons which encode the mutated HPPD according to the invention have been optimized in accordance with the host organism in which it is to be expressed (e.g., by replacing codons with those codons more preferred or most preferred in codon usage tables of such host organism or the group to which such host organism belongs, compared to the original host), with these methods of optimization being well known to the skilled person.

An "isolated DNA", as used herein, refers to a DNA which is not naturally-occurring or no longer in the natural environment wherein it was originally present, e.g., a DNA coding sequence associated with other regulatory elements in a chimeric gene, a DNA transferred into another host cell, such as a plant cell, or an artificial, synthetic DNA having a different nucleotide sequence compared to any known naturally-occurring DNA."

The sequence which encodes an original unmutated HPPD which will be mutated according to the invention, can be of any origin whatever. In particular, it can be of bacterial origin. Advantageous examples which may be cited are bacteria of the *Pseudomonas sp.* type, for example *Pseudomonas fluorescens,* or otherwise cyanobacteria of the *Synechocystis* genus. The sequence can also be of plant origin, in particular derived from dicotyledonous plants, umbelliferous plants, or otherwise monocotyledonous plants. Advantageous examples which may be cited are plants such as tobacco, *Arabidopsis, Daucus carotta*, *Zea mais* (corn), wheat, barley, *Avena sativa*, wheat, *Brachiaria platyphylla*, *Cenchrus echinatus, Lolium rigidum, Festuca arundinacea, Setaria faberi*, *Eleusine indica*, and Sorghum. The coding sequences, and the way of isolating and cloning them, are described in the previously cited references. In a particular embodiment of the invention, the HPPD is from a bacterial origin, particularly from *Pseudomonas* sp., *more particularly from Pseudomonas fluorescens,* or from a plant origin, particularly from *Arabidopsis thaliana.*
The HPPD to make the mutation (s) in for the purpose of the invention, can be any naturally-occurring HPPD, or any active fragment thereof or any variant thereof wherein some amino acids (1 to 10 amino acids) have been replaced, added or deleted for cloning purposes, to make a transit peptide fusion, and the like, which retains HPPD activity, i.e. the property of catalysing the conversion of para-hydroxyphenylpyruvate to homogentisate.

According to the invention, the HPPD may be a chimeric HPPD. The term "chimeric HPPD" is intended to mean an HPPD comprising elements originating from various HPPDs. Such chimeric HPPDs are in particular described in patent application WO 99/24586.

The mutation can be effected in the nucleic acid sequence which encodes the original unmutated HPPD by any means which is appropriate for replacing, in the said sequence, the codon which encodes the mutated amino acid with the codon which corresponds to the amino acid which is to replace it, with the said codons being widely described in the literature and well known to the skilled person.

Several molecular biological methods can be used to achieve this mutation.

A preferred method for preparing a mutated nucleic acid sequence according to the invention, and the corresponding protein, comprises carrying out site-directed mutagenesis on codons encoding one or more amino acids which are selected in advance, including the codon for reference position Gly336 with reference to the Pseudomonas HPPD sequence of SEQ ID NO:2. The methods for obtaining these site-directed mutations are well known to the skilled person and widely described in the literature (in particular: Directed Mutagenesis: A Practical Approach, 1991, Edited by M.J. McPHERSON, IRL PRESS), or are methods for which it is possible to employ commercial kits (for example the U.S.E. mutagenesis kit from PHARMACIA). After the site-directed mutagenesis, it is useful to select the cells which contain a mutated HPPD which is less sensitive to an HPPD inhibitor by using an appropriate screening aid. One screening method which is simple to implement is to determine the dose of HPPD inhibitor which fully inhibits the original unmutated HPPD, and which is lethal for the cells which express this unmutated HPPD, and to subject the mutated cells to this predetermined dose, and thereafter to isolate the mutated cells which have withstood this lethal dose, and then to isolate and to clone the gene which encodes the mutated HPPD. In view of a particular embodiment of the invention and the sought-after solution, i.e. an HPPD which is less sensitive to a triketone, the screening may be performed as described above using a triketone or a pyrazolinate HPPD inhibitor, particularly tembotrione.

In view of another embodiment of the invention, i.e. an HPPD which is further mutated on a second amino acid, in addition to the first mutation on the reference amino acid in position 336 with reference to the Pseudomonas HPPD sequence of SEQ ID NO:2, the second mutation may be obtained by site-directed mutagenesis, performed simultaneously or successively to the first one.

As an alternative to the site-directed mutagenesis as described above, the second mutation may be obtained using methods of random mutation (such as EMS or radiation treatment)associated with an appropriate screening aid. Such methods of mutation are well known to the skilled person, and are amply described in the literature (in particular: Sambrook et al., 1989). Screening methods can be performed as described above.

The terminology DNA or protein "comprising" a certain sequence X, as used throughout the text, refers to a DNA or protein including or containing at least the sequence X, so that other nucleotide or amino acid sequences can be included at the 5' (or N-terminal) and/or 3' (or C-terminal) end, e.g. (the nucleotide sequence of) a selectable marker protein, (the nucleotide sequence of) a transit peptide, and/or a 5' leader sequence or a 3' trailer sequence. Similarly, use of the term "comprise", "comprising" or "comprises" throughout the text and the claims of this application should be understood to imply the inclusion of a stated integer or step or group of integers or steps but not the exclusion of any other integer or step or group of integers or steps

The present invention therefore also relates to a method for preparing a nucleic acid sequence which encodes a mutated HPPD according to the invention, with the said method being defined above.

The invention also relates to the use, in a method for transforming plants, of a nucleic acid which encodes a mutated HPPD according to the invention as a marker gene or as a coding sequence which makes it possible to confer to the plant tolerance to herbicides which are HPPD inhibitors, and the use of HPPD inhibitors on plants comprising a nucleic acid sequence encoding a mutated HPPD according to the invention. In an embodiment of this invention, in such use the HPPD inhibitors are triketones or pyrazolinates, preferably tembotrione, mesotrione or sulcotrione. It is of course understood that this sequence can also be used in combination with (an)other gene marker(s) and/or sequence(s) which encode(s) one or more protein with useful agricultural properties.
Among the genes which encode proteins that confer useful agronomic properties on the transformed plants, mention can be made of the DNA sequences encoding proteins which confer tolerance to certain herbicides, those which confer tolerance to certain insects, those which confer tolerance to certains diseases, etc... Such genes are in particular described in Patent Applications WO 91/02071 and WO95/06128. Among the DNA sequences encoding proteins which confer tolerance to certain herbicides on the transformed plant cells and plants, mention can be made of the bar gene which confers tolerance to glufosinate herbicides, the gene encoding a suitable EPSPS which confers tolerance to herbicides having EPSPS as a target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5,633,435), the gene encoding glyphosate oxydoreductase (US 5,463,175).
Among the DNA sequences encoding a suitable EPSPS which confer tolerance to the herbicides which have EPSPS as a target, mention will more particularly be made of the gene which encodes a plant EPSPS, in particular maize EPSPS, which has two mutations, 102 and 106, and which is described in Patent Application FR 2 736 926, hereinafter named EPSPS double mutant, or the gene which encodes an EPSPS isolated from agrobacterium and which is described by sequence ID No. 2 and sequence ID No. 3 of US Patent 5,633,435, hereinafter named CP4.

In the cases of the DNA sequences encoding EPSPS, and more particularly encoding the genes above, the sequence encoding these enzymes is advantageously preceded by a sequence encoding a transit peptide, in particular encoding the "optimized transit peptide" described in US Patent 5,510,471 or 5,633,448.

Among the DNA sequences encoding proteins of interest which confer novel properties of tolerance to insects, mention will more particularly be made of the Bt proteins widely described in the literature and well known to those skilled in the art. Mention will also be made of proteins extracted from bacteria such as Photorhabdus (WO 97/17432 & WO 98/08932).

The present invention also relates to a chimeric gene (or expression cassette) which comprises a coding sequence as well as heterologous regulatory elements, at the 5' and/or 3' position, at least at the 5' position, which are able to function in a host organism, in particular plant cells or plants, with the coding sequence containing at least one nucleic acid sequence which encodes a mutated HPPD as previously defined.
The present invention therefore relates to a chimeric gene (or expression cassette) which comprises a coding sequence as well as heterologous regulatory elements, at the 5' and/or 3' position, at least at the 5' position, which are able to function in a host organism, in particular plant cells or plants, with the coding sequence containing at least one nucleic acid sequence as previously defined.

In a particular embodiment, the present invention relates to a chimeric gene as previously described, wherein the host organism is selected from bacteria, yeasts, *Pichia*, fungi, baculovirus, plant cells and plants.

In another particular embodiment, the present invention relates to a chimeric gene as previously described, wherein the chimeric gene contains in the 5' position of the nucleic acid sequence which encodes a mutated HPPD, a nucleic acid sequence which encodes a plant transit peptide, with this sequence being arranged between the promoter region and the sequence encoding the mutated HPPD so as to permit expression of a transit peptide/mutated HPPD fusion protein.

As a regulatory sequence which is a promoter in plant cells and plants, use may be made of any promoter sequence of a gene which is naturally expressed in plants, in particular a promoter which is expressed especially in the leaves of plants, such as for example "constitutive" promoters of bacterial, viral or plant origin, or "light-dependent" promoters, such as that of a plant ribulose-biscarboxylase/oxygenase (RuBisCO) small subunit gene, or any suitable known promoter which may be used. Among the promoters of plant origin, mention will be made of the histone promoters as described in Application EP 0 507 698, or the rice actin promoter (US 5,641,876). Among the promoters of a plant virus gene, mention will be made of that of the cauliflower mosaic virus (CAMV 19S or 35S), or the circovirus promoter (AU 689 311).

Use may also be made of a regulatory promoter sequence specific for particular regions or tissues of plants, such as promoters specific for seeds (Datla, R. et al., 1997), especially the napin promoter (EP 255 378), the phaseolin promoter, the glutenin promoter, the helianthinin promoter (WO 92/17580), the albumin promoter (WO 98/45460), the oleosin promoter (WO 98/45461), the SAT1 promoter or the SAT3 promoter (PCT/US98/06978).

Use may also be made of an inducible promoter advantageously chosen from the phenylalanine ammonia lyase (PAL), HMG-CoA reductase (HMG), chitinase, glucanase, proteinase inhibitor (PI), PR1 family gene, nopaline synthase (nos) and vspB promoters (US 5 670 349, Table 3), the HMG2 promoter (US 5 670 349), the apple beta-galactosidase (ABG1) promoter and the apple aminocyclopropane carboxylate synthase (ACC synthase) promoter (WO 98/45445).

According to the invention, use may also be made, in combination with the promoter, of other regulatory sequences, which are located between the promoter and the coding sequence, such as transcription activators ("enhancers"), for instance the translation activator of the tobacco mosaic virus (TMV) described in Application WO 87/07644, or of the tobacco etch virus (TEV) described by Carrington & Freed 1990, for example, or introns such as the adh1 intron of maize or intron 1 of rice actin.

As a regulatory terminator or polyadenylation sequence, use may be made of any corresponding sequence of bacterial origin, such as for example the nos terminator of Agrobacterium tumefaciens, of viral origin, such as for example the CaMV 35S terminator, or of plant origin, such as for example a histone terminator as described in Application EP 0 633 317.

"Host organism" is understood as being any unicellular or multicellular organism into which the chimeric gene according to the invention can be introduced for the purpose of producing mutated HPPD. These organisms are, in particular, bacteria, for example *E. coli*, yeasts, in particular of the genera *Saccharomyces* or *Kluyveromyces*, *Pichia*, fungi, in particular *Aspergillus*, a baculovirus or, preferably, plant cells and plants.

"Plant cell" is understood, according to the invention, as being any cell which is derived from or found in a plant and which is able to form or is part of undifferentiated tissues, such as calli, differentiated tissues such as embryos, parts of plants, plants or seeds.

"Plant" is understood, according to the invention, as being any differentiated multicellular organism which is capable of photosynthesis, in particular a monocotyledonous or dicotyledonous organism, more especially cultivated plants which are or are not intended for animal or human nutrition, such as maize or corn, wheat, Brassica spp. plants such as Brassica napus or Brassica juncea, soybean, rice, sugarcane, beetroot, tobacco, cotton, vegetable plants such as cucumber, leek, carrot, tomato, lettuce, peppers, melon, watermelon, etc.

In one embodiment the invention relates to the transformation of plants. Any promoter sequence of a gene which is expressed naturally in plants, or any hybrid or combination of promoter elements of genes expressed naturally in plants, including Agrobacterium or plant virus promoters, or any promoter which is suitable for controlling the transcription of a herbicide tolerance gene, can be used as the promoter regulatory sequence in the plants of the invention. Examples of such suitable promoters are described above.

According to the invention, it is also possible to use, in combination with the promoter regulatory sequence, other regulatory sequences which are located between the promoter and the coding sequence, such as intron sequences, or transcription activators (enhancers). Examples of such suitable regulatory sequences are described above.
Any corresponding sequence of bacterial origin, such as the nos terminator from *Agrobacterium tumefaciens*, or of plant origin, such as a histone terminator as described in application EP 0 633 317, may be used as transcription termination (and polyadenylation) regulatory sequence.

In one particular embodiment of the invention, a nucleic acid sequence which encodes a transit peptide is employed 5' of the nucleic acid sequence encoding a mutated HPPD, with this transit peptide sequence being arranged between the promoter region and the sequence encoding the mutated HPPD so as to permit expression of a transit peptide/mutated HPPD fusion protein, with the mutated HPPD being previously defined. The transit peptide makes it possible to direct the mutated HPPD into the plastids, more especially the chloroplasts, with the fusion protein being cleaved between the transit peptide and the mutated HPPD when the latter enters the plastid. The transit peptide may be a single peptide, such as an EPSPS transit peptide (described in US patent 5,188,642) or a transit peptide of that of the plant ribulose biscarboxylase/ oxygenase small subunit (RuBisCO ssu), where appropriate including a few amino acids of the N-terminal part of the mature RuBisCO ssu (EP 189 707), or else may be a fusion of several transit peptides such as a transit peptide which comprises a first plant transit peptide which is fused to a part of the N-terminal sequence of a mature protein having a plastid location, with this part in turn being fused to a second plant transit peptide as described in patent EP 508 909, and, more especially, the optimized transit peptide which comprises a transit peptide of the sunflower RuBisCO ssu fused to 22 amino acids of the N-terminal end of the maize RuBisCO ssu, in turn fused to the transit peptide of the maize RuBisCO ssu, as described, with its coding sequence, in patent EP 508 909.

The present invention also relates to the transit peptide/mutated HPPD fusion protein and a nucleic acid or plant-expressible chimeric gene encoding such fusion protein, wherein the two elements of this fusion protein are as defined above.

The present invention also relates to a cloning and/or expression vector for transforming a host organism, which vector contains at least one chimeric gene as defined above. In addition to the above chimeric gene, this vector contains at least one origin of replication. This vector can be a plasmid, a cosmid, a bacteriophage or a virus which has been transformed by introducing the chimeric gene according to the invention. Such transformation vectors, which depend on the host organism to be transformed, are well known to the skilled person and widely described in the literature. The transformation vector which is used, in particular, for transforming plant cells or plants may be a virus, which can be employed for transforming developed plants and which additionally contains its own replication and expression elements. According to the invention, the vector for transforming plant cells or plants is preferably a plasmid, such as a disarmed *Agrobacterium* Ti plasmid.

The present invention also relates to the host organisms, in particular plant cells or plants, which are transformed and which contain a chimeric gene which comprises a sequence encoding a mutated HPPD as defined above, and the use of the plants of the invention in a field to grow a crop and harvest a plant product, e.g., soybean or corn grains, where in one embodiment said use involves the application of HPPD inhibitor herbicides to such plants to control weeds. In one embodiment of this invention, in such use the HPPD inhibitors are triketones or pyrazolinates, preferably tembotrione, mesotrione or sulcotrione, particularly tembotrione.

Therefore, the present invention relates to a host organism, in particular a plant cell or plant, characterized in that it contains at least one chimeric gene as previously described above, or at least a nucleic acid sequence as previously described.

In a particular embodiment, the present invention relates to a plant cell or plant characterized in that it contains at least a nucleic acid sequence which encodes a mutated HPPD enzyme which retain its properties of catalysing the conversion of para-hydroxyphenylpyruvate (HPP) to homogentisate and which is less sensitive to an HPPD inhibitor than the original unmutated HPPD, characterized in that it contains a mutation at the position 336 (amino acid glycine in the native HPPD) with reference to the *Pseudomonas* HPPD of SEQ ID NO:2 which is Gly336Phe. In a further more particular embodiment, the present invention relates to a plant cell or plant characterized in that it contains at least a nucleic acid sequence which encodes a mutated HPPD as described above, wherein the mutation in the position 336 with reference to the *Pseudomonas* HPPD of SEQ ID NO:2 is Gly336Phe.

In another particular embodiment, the present invention relates to a plant cell or plant characterized in that it contains at least a nucleic acid sequence which encodes a mutated HPPD which retain its properties of catalysing the conversion of para-hydroxyphenylpyruvate (HPP) to homogentisate and which is less sensitive to an HPPD inhibitor than the original unmutated HPPD, wherein the HPPD enzyme is from a plant and contains a mutation on glycine at position 422 with reference to the amino acid sequence of the *Arabidopsis* HPPD of SEQ ID NO:4 (i.e. position 336 with reference to the amino acid sequence of the Pseudomonas HPPD of SEQ ID NO:2) which is Gly336Phe.

In a more particular embodiment, the present invention relates to a plant cell or plant characterized in that it contains at least a nucleic acid sequence which encodes a mutated HPPD as described above, wherein the mutated HPPD is less sensitive than the original unmutated HPPD to tembotrione.

In another particular embodiment, the present invention relates to a plant cell or plant characterized in that it contains at least a nucleic acid sequence which encodes a mutated HPPD as described above, wherein the mutated HPPD is less sensitive to tembotrione than the original unmutated HPPD.

In a more particular embodiment, the present invention relates to a plant cell or plant characterized in that it contains at least a nucleic acid sequence which encodes a mutated HPPD as described above, wherein the mutated HPPD is less sensitive to tembotrione.

In another particular embodiment, the present invention relates to a plant cell or plant characterized in that it contains at least a nucleic acid sequence which encodes a mutated HPPD as described above, wherein the mutated HPPD of the invention contains a second mutation, in addition to the first mutation on the amino acid glycine at the position 336 with reference to the *Pseudomonas* HPPD of SEQ ID NO:2.

In a more particular embodiment, the present invention relates to a plant cell or plant characterized in that it contains at least a nucleic acid sequence which encodes a mutated HPPD as described above, wherein the second mutated amino acid is selected from the selected amino acids: Pro215, Gly298, Gly332, Phe333, Gly334 and Asn337, with reference to the *Pseudomonas* HPPD sequence of SEQ ID NO:2.

In a more particular embodiment, the present invention relates to a plant cell or plant characterized in that it contains at least a nucleic acid sequence which encodes a mutated HPPD enzyme which is less sensitive to the HPPD inhibitor tembotrione of the class of triketones than the original unmutated HPPD and is mutated in the position 336 with reference to the *Pseudomonas* HPPD of SEQ ID NO:2 according to the mutation Gly336Phe.

In another particular embodiment, the present invention relates to a plant cell or plant characterized in that it contains at least a nucleic acid sequence as previously described, and in addition a gene that is functional in plants, allowing overexpression of a PDH (prephenate dehydrogenase) enzyme.

The present invention also relates to the plants which contain transformed cells, in particular the plants which are regenerated from the transformed cells. The regeneration can be obtained by any appropriate method, with the method depending on the nature of the species, as described, for example, in the above references. The following patents and patent applications may be cited, in particular, with regard to the methods for transforming plant cells and regenerating plants: US 4,459,355, US 4,536,475, US 5,464,763, US 5,177,010, US 5,187,073, EP 267,159, EP 604 662, EP 672 752, US 4,945,050, US 5,036,006, US 5,100,792, US 5,371,014, US 5,478,744, US 5,179,022, US 5,565,346, US 5,484,956, US 5,508,468, US 5,538,877, US 5,554,798, US 5,489,520, US 5,510,318, US 5,204,253, US 5,405,765, EP 442 174, EP 486 233, EP 486 234, EP 539 563, EP 674 725, WO 91/02071 and WO 95/06128.

The present invention also relates to the transformed plants or part thereof, which are derived by cultivating and/or crossing the above regenerated plants, and to the seeds of the transformed plants.

The present disclosure also relates to the end products such as the meal or oil which are obtained from the plants, part thereof, or seeds of the invention.

The transformed plants which can be obtained in accordance with the invention can be of the monocotyledonous type, such as cereals, sugarcane, rice and corn or maize, or of the dicotyledonous type, such as tobacco, soybean, Brassica spp. plants such as oilseed rape, cotton, beetroot, clover, etc.

The invention relates to a method for transforming host organisms, in particular plant cells or plants, by integrating in such organisms at least one nucleic acid sequence or one chimeric gene as previously defined, wherein it is possible to obtain the transformation by any appropriate known means, which means are amply described in the specialist literature and, in particular, the references cited in the present application, more especially by using the vector according to the invention.

One series of methods comprises bombarding cells, protoplasts or tissues with particles to which the DNA sequences are attached. Another series of methods comprises using, as the means for transfer into the plant, a chimeric gene which is inserted into an Agrobacterium tumefaciens Ti plasmid or an Agrobacterium rhizogenes Ri plasmid. Other methods may be used, such as microinjection or electroporation or otherwise direct precipitation using PEG. The skilled person can select any appropriate method for transforming the host organism of choice, in particular the plant cell or the plant. As examples, the technology for soybean transformation has been extensively described in the examples 1 to 3 of EP 1186666. For rice, agrobacterium-mediated transformation (Hiei et al., 1994, and Hiei et al., 1997), electroporation (US Patent 5,641,664 and US Patent 5,679,558), or bombardment (Christou et al., 1991)could be performed. A suitable technology for transformation of monocotyledonous plants, and particularly rice, is described in WO 92/09696. For cotton, agrobacterium-mediated transformation (Gould J.H. and Magallanes-Cedeno M., 1998 and Zapata C.,1999), polybrene and/or treatment-mediated transformation (Sawahel W.A.,2001) have been described.

In a particular embodiment of the invention, the mutated HPPD is targeted into the chloroplast. This may be done by integrating a nucleic acid sequence which encodes a transit peptide/mutated HPPD fusion protein as described above.

Alternatively, the mutated HPPD may be expressed directly in the chloroplasts using transformation of the chloroplast genome. A suitable method comprises the bombardment of leaf sections by particles coated with the DNA and integration of the introduced gene encoding the protein of the invention by homologous recombination. Suitable vectors and selection systems are known to the person skilled in the art. An example of means and methods which can be used for such integration into the chloroplast genome of tobacco lines is given in WO 06/108830. When the polypeptides are directly targeted to the chloroplast using transformation of the chloroplast genome, a transit peptide sequence is generally not required.

The present invention also relates to a method for obtaining a plant resistant to an HPPD inhibitor, characterized in that the plant is transformed with a chimeric gene as previously described.

Therefore, the present invention also relates to a method for obtaining a plant resistant to an HPPD inhibitor, characterized in that the plant is transformed with a chimeric gene which comprises a coding sequence as well as heterologous regulatory element in the 5' and optionally in the 3' positions, which are able to function in a host organism, characterized in that the coding sequence contains at least a nucleic acid sequence as previously described.

In a particular embodiment of this invention, in this method the HPPD inhibitor is a triketone, or preferably tembotrione, mesotrione or sulcotrione, particularly tembotrione.

In another particular embodiment, the present invention relates to a method for obtaining a plant resistant to an HPPD inhibitor as described above, characterized in that the plant is further transformed, simultaneously or successively, with a gene functional in this plant allowing overexpression of a PDH (prephenate dehydrogenase) enzyme.

The invention also relates to a method for selectively weeding plants, in particular plant crops, with the aid of an HPPD inhibitor, in particular a herbicide as previously defined, which method is characterized in that this herbicide is applied to plants which have been transformed in accordance with the invention, either before sowing the crop, before emergence of the crop or after emergence of the crop.

In a particular embodiment of this invention, in this method the HPPD inhibitor is a triketone preferably tembotrione, mesotrione or sulcotrione, particularly tembotrione.

The invention also relates to a method for controlling weeds in an area or a field which contains transformed seeds as previously described in the present patent application, which method comprises applying, to the said area of the field, a dose of a HPPD inhibitor herbicide which is toxic for the said weeds, without significantly affecting the seeds or plants which contains a nucleic acid sequence or a chimeric gene as previously described in the present patent application.

In a particular embodiment of this invention, in this method the HPPD inhibitor is a triketone, or preferably tembotrione, mesotrione or sulcotrione, particularly tembotrione.

The present invention also relates to a method for cultivating the plants which have been transformed with a chimeric gene according to the invention, which method comprises planting seeds comprising a chimeric gene of the invention, in an area of a field which is appropriate for cultivating the said plants, and in applying, if weeds are present, a dose, which is toxic for the weeds, of a herbicide whose target is the above-defined HPPD to the said area of the said field, without significantly affecting the said transformed seeds or the said transformed plants, and in then harvesting the cultivated plants or plant parts when they reach the desired stage of maturity and, where appropriate, in separating the seeds from the harvested plants.

In a particular embodiment of this invention, in this method the HPPD inhibitor is a triketone, or preferably tembotrione, mesotrione or sulcotrione, particularly tembotrione.

In the above methods, the herbicide whose target is the HPPD can be applied in accordance with the invention, either before sowing the crop, before the crop emerges or after the crop emerges.

The present invention also relates to a process for obtaining oil, particularly soybean oil, or meal, comprising growing a crop, particularly a soybean crop, expressing a mutated HPPD of the invention in a field, optionally treating such crop with an HPPD inhibitor herbicide, harvesting the grains and milling the grains to make meal and extract the oil. Also the plants seeds or grains, either whole, broken or crushed, containing the chimeric gene of the invention are part of this invention.

Therefore, the present invention relates to a method for obtaining oil or meal comprising growing a transformed plant as described above, optionally treating such plant with an HPPD inhibitor herbicide, harvesting the grains and milling the grains to make meal and extract the oil.

In particular embodiments, the above methods of the invention are involving an HPPD inhibitor herbicide selected from isoxaflutole, tembotrione, mesotrione, pyrasulfotole, sulcotrione, topramezone, 2-cyano-3-cyclopropyl-1-(2-SO₂CH₃-4-CF₃phenyl)propane-1,3-dione and 2-cyano-3-cyclopropyl-1-(2-SO₂CH₃-4-2,3 Cl₂ phenyl)propane-1,3-dione.

In other particular embodiments, the above methods of the invention are involving an HPPD inhibitor herbicide of the class of triketones, such as tembotrione, sulcotrione and mesotrione, particularly tembotrione.

Within the meaning of the present disclosure "herbicide" is understood as being a herbicidally active substance on its own or such a substance which is combined with an additive which alters its efficacy, such as, for example, an agent which increases its activity (a synergistic agent) or which limits its activity (a safener) . It is of course to be understood that, for their application in practice, the above herbicides are combined, in a manner which is known per se, with the formulation adjuvants which are customarily employed in agricultural chemistry.

When the plant which has been transformed in accordance with the invention contains one or more other genes for tolerance towards other herbicides (as, for example, a gene which encodes a mutated or unmutated EPSPS which confers on the plant tolerance to glyphosate herbicides or a pat or bar gene conferring tolerance to glufosinate herbicides), or when the transformed plant is naturally sensitive to another herbicide (such as sulfonylurea tolerance), the method according to the invention can comprise the simultaneous or chronologically staggered application of an HPPD inhibitor in combination with the said herbicide or herbicide combination, for example glyphosate and/or glufosinate and/or sulfonylurea herbicides.

The invention also relates to the use of the chimeric gene encoding a mutated HPPD according to the invention as a marker gene during the transformation of a plant species, based on the selection on the abovementioned HPPD inhibitor herbicides.

The present disclosure also relates to a method for obtaining a plant resistant to a triketone characterized in that the plant is transformed with a chimeric gene expressing in the plant a HPPD mutated in the amino acid glycine at position 336 with reference to the amino acid sequence of the *Pseudomonas* HPPD of SEQ ID NO: 2 which is Gly336Phe.

In a particular embodiment, the invention relates to said method for obtaining a plant resistant to a triketone HPPD inhibitor, characterized in that the HPPD mutation is Gly336Phe.

In another particular embodiment, the invention relates to said method for obtaining a plant resistant to a triketone HPPD inhibitor selected from tembotrione, mesotrione and sulcotrione.

In another particular embodiment, the invention relates to said method for obtaining a plant resistant to a triketone HPPD inhibitor, characterized in that the plant is further transformed, simultaneously or successively, with a gene functional in this plant allowing overexpression of a PDH (prephenate dehydrogenase) enzyme.

The invention also relates to a method for controlling weeds in an area or a field, which method comprises planting in this area or field transformed plants resistant to a triketone HPPD inhibitor which has been obtained according to the method described above, or transformed seeds which originates from them, and in applying a dose which is toxic for the weeds of said triketone HPPD inhibitor without significantly affecting the said transformed seeds or the said transformed plants.

The invention also relates to a method for obtaining oil or meal comprising growing a transformed plant resistant to a triketone HPPD inhibitor which has been obtained according to the method described above, or a transformed seed which originates from such plant, optionally treating such plant or seed with a triketone HPPD inhibitor, harvesting the grains and milling the grains to make meal and extract the oil. The invention also relates to the use of a HPPD which has been mutated in the amino acid glycine at the position 336 with reference to the amino acid sequence of the *Pseudomonas* HPPD of SEQ ID NO:2 which is Gly336Phe to render plants tolerant to a triketone HPPD inhibitor.

The invention also relates to the use of a mutated HPPD as described above, characterized in that the HPPD inhibitor is a triketone HPPD inhibitor selected from tembotrione, mesotrione, and sulcotrione.

The present invention also relates to a host organism, in particular plant cells or plants, which contain a chimeric gene comprising a sequence encoding a mutated HPPD according to the invention, and which also contain a gene functional in this host organism allowing overexpression of a prephenate dehydrogenase (abbreviated herein as PDH) enzyme. In the expression "gene that is functional in plants, allowing overexpression of a PDH enzyme", the term "PDH" should be interpreted as referring to any natural or mutated PDH enzyme exhibiting the PDH activity of conversion of prephenate to HPP. In particular, said PDH enzyme can originate from any type of organism. An enzyme with PDH activity can be identified by any method that makes it possible either to measure the decrease in the amount of prephenate substrate, or to measure the accumulation of a product derived from the enzymatic reaction, i.e. HPP or one of the cofactors NADH or NADPH. In particular, the PDH activity can be measured by means of the method described in example 4. Many genes encoding PDH enzymes are described in the literature, and their sequences can be identified on the website http://www.ncbi.nlm.nih.gov/entrez/. Particularly known is the gene encoding the PDH enzyme of the yeast *Saccharomyces cerevisiae* (Accession No. S46037) as described in Mannhaupt et al. (1989), of a bacterium of the *Bacillus* genus, in particular of the species *B. subtilis* (Accession No. P20692) as described in Henner et al. (1986), of a bacterium of the *Escherichia* genus, in particular of the species *E*. *coli* (Accession No. KMECTD) as described in Hudson et al. (1984), or of a bacterium of the *Erwinia* genus, in particular of the species *E*. *herbicola* (Accession No. S29934) as described in Xia et al. (1992). The invention further relates to a method for obtaining a host organism, particularly a plant cell or a plant, resistant to an HPDD inhibitor by integrating in such organism at least one nucleic acid sequence or one chimeric gene as defined above, and by further transforming it, simultaneously or successively, with a gene functional in this host organism allowing overexpression of a PDH (prephenate dehydrogenase) enzyme.

In a particular embodiment, the invention relates to a method for obtaining a host organism, particularly a plant cell or a plant, resistant to a triketone HPDD inhibitor, particularly tembotrione, mesotrione or sulcotrione.

Means and methods which could be used for obtaining a host organisms, particularly a plant cell or a plant, transformed both with a gene allowing overexpression of an HPPD enzyme, and with a gene allowing overexpression of a PDH enzyme are extensively described in WO 04/024928.

The reference in this specification to any prior publication (or information derived from it), or to any matter which is known, is not, and should not be taken as an acknowledgement or admission or any form of suggestion that that prior publication (or information) or known matter forms part of the common general knowledge in the field of this invention.

### FIGURES

Fig.1: Alignment the HPPD sequences of *Streptomyces avermitilis*, *Daucus carota*, *Arabidopsis thaliana*, *Zea mais*, *Hordeum vulgare*, *Mycosphaerella graminicola*, *Coccicoides immitis*, *Mus musculus*, and *Pseudomonas fluorescens.* The numbering of the amino acids is done according to the *Pseudomonas* sequence, and an asterisk designates the amino acids which are common to these sequences.

### SEQUENCES LISTING

SEQ ID NO 1: Nucleic acid sequence encoding *Pseudomonas fluorescens* HPPD
SEQ ID NO 2: *Pseudomonas fluorescens* HPPD amino acid sequence
SEQ ID NO 3: Nucleic acid sequence encoding *Arabidopsis thaliana* HPPD
SEQ ID NO 4: *Arabidopsis thaliana* HPPD amino acid sequence
SEQ ID NO 5: Nucleic acid sequence encoding *Mus musculus* HPPD
SEQ ID NO 6: *Mus musculus* HPPD amino acid sequence SEQ ID NO 7: Nucleic acid sequence encoding *Coccidioides immitis* HPPD
SEQ ID NO 8: *Coccidioides immitis* HPPD amino acid sequence
SEQ ID NO 9: Nucleic acid sequence encoding *Mycosphaerella graminicola* HPPD
SEQ ID NO 10: *Mycosphaerella graminicola* HPPD amino acid sequence
SEQ ID NO 11: Nucleic acid sequence encoding *Hordeum vulgare* HPPD
SEQ ID NO 12: *Hordeum vulgare* HPPD amino acid sequence
SEQ ID NO 13: Nucleic acid sequence encoding *Zea mais* HPPD
SEQ ID NO 14: *Zea mais* HPPD amino acid sequence SEQ ID NO 15: Nucleic acid sequence encoding *Daucus carota* HPPD
SEQ ID NO 16: *Daucus carota* HPPD amino acid sequence SEQ ID NO 17: Nucleic acid sequence encoding *Streptomyces avermitilis* HPPD
SEQ ID NO 18: *Streptomyces avermitilis* HPPD amino acid sequence
SEQ ID NO 19: primer sequence kerfi001
SEQ ID NO 20: primer sequence kerfi002
SEQ ID NO 21: primer sequence kerfi003
SEQ ID NO 22: primer sequence kerfi004
SEQ ID NO 23: primer sequence kerfi007
SEQ ID NO 24: primer sequence kerfi008
SEQ ID NO 25: primer sequence kerfi011
SEQ ID NO 26: primer sequence kerfi012
SEQ ID NO 27: primer sequence kerfi014
SEQ ID NO 28: primer sequence kerfi016
SEQ ID NO 29: primer sequence kerfi019
SEQ ID NO 30: primer sequence kerfi020
SEQ ID NO 31: primer sequence kerfi015
SEQ ID NO 32: primer sequence kerfi018

### EXAMPLES

The various aspects of the invention will be better understood with the aid of the experimental examples which follow. All the methods or operations which are described below in these examples are given by way of example and correspond to a choice which is made from among the different methods which are available for arriving at the same or similar result. This choice has no effect on the quality of the result and, as a consequence, any suitable method can be used by the skilled person to arrive at the same or similar result. The majority of the methods for manipulating DNA fragments are described in "Current Protocols in Molecular Biology" Volumes 1 and 2, Ausubel F.M. et al., published by Greene Publishing Associates and Wiley Interscience (1989) or in Molecular cloning, T. Maniatis, E.F. Fritsch, J. Sambrook, 1982, or in Sambrook J. and Russell D., 2001, Molecular Cloning: a laboratory manual (Third edition)

### Example 1: Preparation of mutated HPPD

### General outline

The *Arabidopsis thaliana At*HPPD coding sequence (1335 bp)(Genebank AF047834; WO 96/38567) was initially cloned into the expression vector pQE-30 (QIAGEN) in between the restriction sites of BamHI and HindIII.

The *Pseudomonas fluorescens Pf*HPPD coding sequence (1174 bp) (Rüetschi et al., Eur. J. Biochem., 205, 459-466, 1992, WO 96/38567) was initially cloned into the unique NcoI site of the expression vector pKK233-2 (Pharmacia) that provides a start codon.

The vectors pQE-30-*AtHPPD* and pKK233-2-*PfHPPD* were used for PCR-mediated attachment of an *Nco*I restriction site and of a sequence encoding an N-terminal His₆-Tag to the 5' ends and an *XbaI* restriction site to the 3' ends of *AtHPPD* and *PfHPPD.*

The PCR product of the *AtHPPD* gene was isolated from an agarose gel, cut with the restriction enzymes *Nco*I and *Xba*I, purified with the MinElute™ PCR Purification Kit (Qiagen) and cloned into the pSE420(RI)NX vector cut with the same restriction enzymes.

Concerning the *PfHPPD* gene, the PCR product was isolated from an agarose gel and cloned into the pCR®2.1-TOPO® vector. It was excised from this vector with the restriction enzymes *Nco*I and *Xba*I, isolated from an agarose gel and cloned into the pSE420(RI)NX vector cut with the same restriction enzymes.

Both pSE420(RI)NX-*AtHPPD* and *-PfHPPD* were then subjected to PCR-mediated site-directed mutagenesis to alter a defined codon at corresponding sites of both genes. The respective codon encodes Gly336 in WT *Pf*HPPD and Gly422 in WT *At*HPPD.
The mutated codons in the coding sequences are analyzed using the Pyrosequencing® technique.

### PCR-mediated attachment of a sequence encoding an N-terminal His₆-tag and NcoI and XbaI restriction sites:

The PCR reaction for each gene (*AtHPPD* and *PfHPPD*) was carried out in 24 wells of a 96 well PCR plate, respectively. Since the forward and reverse primers for this reaction differ in size by 18 *(AtHPPD)* and 22 bp (*PfHPPD*), an annealing temperature gradient from 40.9 °C to 64.5 °C was performed, each well being subjected to another annealing temperature within this range. When the primers anneal to the single stranded template for the first time, a 5' overhang was produced in the new strand until its complementary strand is synthesized and this overhang formed by the 5' region of the first primer is part of the template. The coding sequences were thereby extended at both ends, introducing a sequence encoding a N-terminal His₆-tag and a restriction site at both ends.

The reaction mixtures contain 500 ng of pQE-30-*AtHPPD* DNA (1 µL from plasmid maxipreparation) or 1 µg of pKK233-2-*PfHPPD* DNA (0.75 µL from plasmid maxipreparation), 1 µl of kerfi001 and kerfi002, respectively, for *At*HPPD or kerfi003 and kerfi004, respectively, for *Pf*HPPD (all primer solutions have a concentration of 10 pmol*µL⁻¹), 25 µl HotStarTaq Master Mix (Qiagen)and HyPure™ Molecular Biology Grade Water to a final volume of 50 µL. The PCR programme is set as follows:
1. 95 °C 15 min
2. 94 °C 30 s
   40.9 °C-60.4 °C 30 s
   72 °C 3 min
   Step 2 is repeated 20 times.
3. 72 °C 10 min

| Primer name | Primer sequence |
|---|---|
| kerfi001 | 5'-CCATGGCTCATCACCATCACCATCACCAAAACGCCGCCGTTTCAG-3' |
| kerfi002 | 5'-TCTAGATCATCCCACTAACTGTTTGGC-3' |
| kerfi003 | 5'-CCATGGCTCATCACCATCACCATCACGCAGATCTATACGAAAACCCAATGG-3' |
| kerfi004 | 5'-TCTAGATTAATCGGCGGTCAATACACCAC-3' |

The PCR reactions were subjected to agarose gel electrophoresis which all produced clear bands corresponding to fragments of approximately 1500 bp (*AtHPPD*) or 1100 bp (*PfHPPD*). The bands were excised from the gel and DNA was purified using the QIAquick® Gel Extraction Kit (Qiagen).

### Cloning into pCR®2.1-TOPO® vector (Invitrogen)

pCR®2.1-TOPO® vector (3931 bp) was used for one-step cloning of *Taq* polymerase-amplified PCR products which display a 3'-adenosine (A) overhangs. The vector, in turn, was linearized and displayed single 3'-thymidine (T) overhangs at its ends. Topoisomerase I was covalently attached to these 3'-thymidines which served to covalently link the vector to the PCR product. For selection of bacterial cells carrying the vector, either ampicillin or kanamycin could be used. The vector possessed an *Xba*I restriction site within its multiple cloning site and an *Nco*I restriction site within the KanR gene.

DNA solutions obtained from each gel extraction were used for TOPO TA cloning, respectively. After transformation of *E*. *coli* TOP10 cells, each reaction yielded three white colonies (A1-A3, P1-P3) that were used to inoculate 5 mL LB/amp medium.

To determine whether the vectors of these colonies carried the correct inserted fragment, plasmid DNA was prepared from 4 mL of pCR®2.1-TOPO®-*AtHPPD* cultures A1-A3 and *-PfHPPD* cultures P1-P3 using the QIAprep® Spin Miniprep Kit (Qiagen). DNA solutions obtained from these plasmid preparations were subjected to a restriction digest with *Hind*III and *Xho*I which was then analyzed on a 1 % agarose gel. Both *Hind*III and *Xho*I each possess a single restriction site in the pCR®2.1-TOPO®*-AtHPPD* /*-PfHPPD* vector, respectively. The restriction digest of DNA from clone A1 produced the expected bands representing a 1461 bp fragment (*AtHPPD* coding sequence) and the 3831 bp vector fragment; the restriction digest of P3 produced the expected bands representing a 1206 bp fragment (*PfHPPD* coding sequence) and the 3831 bp vector fragment on the agarose gel.

DNA obtained from plasmid maxipreparation using the QIAfilter™ Maxi Kit (Qiagen) and subsequent NaAc / EtOH precipitation from 100 mL of A1 (*AtHPPD*) or P3 (*PfHPPD*) liquid LB/amp culture was used to determine the DNA sequence of the respective inserted *HPPD* gene in the pCR®2.1-TOPO® vector. DNA sequencing was carried out with the primers M13 uni (-21) and M13 rev (-29) by Eurofins MWG GmbH. Sequencing confirmed the correct DNA sequence of both *AtHPPD* and *PfHPPD* in the pCR®2.1-TOPO® vector, including the restriction sites at both ends of the coding sequences.

### Cloning into pSE420(RI)NX

The cloning and expression vector pSE420(RI)NX (5261 bp) is based on the plasmid pSE420 by Invitrogen. Modifications of this vector include the addition of a kanamycin tolerance gene and the removal of the majority of the superlinker region (multiple cloning site).

The plasmid possesses the trp-lac (trc) promoter and the *lac*I^{q} gene that provides the *lac* repressor in every *E*. *coli* host strain. The *lac* repressor binds to the *lac* operator (*lac*O) and restricts expression of the target gene; this inhibition can be alleviated by induction with Isopropyl β-D-1-thiogalactopyranoside (IPTG) .

The genes *AtHPPD* and *PfHPPD* were cloned into the vector pSE420(RI)NX in between the restriction sites of *NcoI* and *XbaI.*

### PCR-based site-directed mutagenesis:

Template DNA (pSE420(RI)NX-*AtHPPD* and pSE420(RI)NX-*PfHPPD*) were isolated from *E*. *coli* TOP10 liquid culture by performing a plasmid minipreparation. The DNA solutions obtained from these minipreparations were diluted to a concentration of 0.05 µg*µL⁻¹.

PCR-based site-directed mutagenesis requires two chemically synthesized DNA primers (forward and reverse primer) that are complementary to the same DNA region, each of them to one strand of the double-stranded DNA template. These primers contain the desired mutation at their centre and cover a region of about 20-30 nucleotides of the template, including the mutation site and 10-15 bases on each of its sides. The mutation site covers three nucleotides that vary independently in the primers in order to obtain each possible codon at the selected site.
In circular PCR mutagenesis a plasmid template is completely copied by rolling circle replication starting from the 3' OH end of a primer that is incorporated into the growing strand. Each new DNA molecule then carries one or more altered nucleotides that were contained in the primer. A high fidelity DNA polymerase is used in order to reduce the possibility of further undesired mutations.
The oligonucleotide primer pairs kerfi007/kerfi008 (*AtHPPD*) and kerfi011/kerfi012 (*PfHPPD*) were dissolved in water to a concentration of 10 pmol*µL⁻¹. For the mutagenesis PCR reaction, 50 ng of template plasmid from pSE420(RI)NX-*AtHPPD* or pSE420(RI)NX-*PfHPPD* minipreparations, diluted to a concentration of 0.05 µg*µL⁻¹, were used. The reaction mixture was composed as follows:
1 µL template plasmid (0.05 µg*µL⁻¹)
1.5 µL primer kerfi007 (or kerfi011) (10 pmol*µL⁻¹)
1.5 µL primer kerfi008 (or kerfi012) (10 pmol*µL⁻¹)
5 µL 10x reaction buffer
1 µL dNTP mix
40 µL HyPure™ Molecular Biology Grade Water
1 µL *PfuUltra*® High-Fidelity DNA polymerase (2.5 U*µL⁻¹)
The PCR programme was the same for mutagenesis of *AtHPPD* and *PfHPPD* and the elongation time was set to 7 minutes, assuming that it takes 1 minute to replicate 1 kb of plasmid DNA.
1. 95 °C 30 s
2. 95 °C 30 s
   55 °C 30 s
   68 °C 7 min
Step 2 is repeated 18 times.

After the PCR reaction, the reactions were set on ice to cool down to room temperature.

| Primer name | Primer sequence |
|---|---|
| kerfi007 | 5'-GGTGGTTTTGGCAAANNNAATTTCTCTGAGCTC-3' |
| kerfi008 | 5'-GAGCTCAGAGAAATTNNNTTTGCCAAAACCACC-3' |
| kerfi011 | 5'-CAGCGCCTTGAAGTTNNNCTCGCCAAACCCATC-3' |
| kerfi012 | 5'-GATGGGTTTGGCGAGNNNAACTTCAAGGCGCTG-3' |

After the PCR reaction mutant plasmids were selected using the *Dpn* I restriction endonuclease. Only dam-methylated DNA is degraded by the restriction enzyme *Dpn* I whose restriction site G^{Me6}ATC is relatively abundant. Template plasmids which were produced by bacteria have been methylated and are therefore degraded. PCR-amplified DNA, however, remains intact.
1 µL of *Dpn* I restriction enzyme (10 U*µL⁻¹) was added to the PCR reactions and the solutions were mixed by pipetting up and down. After 1 minute of centrifugation (13,200 rpm) the reactions were incubated at 37 °C for 1 hour.

Mutant plasmids contained staggered nicks at the 5' end of each primer and could be directly transformed into competent cells.

To concentrate mutant plasmids, a NaAc / EtOH precipitation was carried out and the DNA was resuspended in 10 µL of HyPure™ Molecular Biology Grade Water. 3 µL of these plasmid solutions were later used for transformation of electro competent *E*. *coli* K-12 MG1655 cells, and, in the case of *AtHPPD*, 1 µL was used for transformation of electro competent *E*. *coli* TOP10 cells.
For *At*HPPD, a total of 62 *E*. *coli* K-12 MG1655 clones were obtained and cultivated for subsequent analysis of the mutated codon in Costar® 96 well 2 mL deep well plates. To obtain higher numbers of clones, *E*. *coli* TOP10 was used as an alternative host for cloning of mutagenized plasmids. Transformation of *E*. *coli* TOP10 cells with mutagenized plasmids yielded several hundreds of clones.
Concerning *Pf*HPPD, a total of 252 *E*. *coli* K-12 MG1655 clones were obtained and cultivated for analysis as described for clones transformed with *AtHPPD* plasmids

### Example 2 : Pyrosequencing® reactions for verifying point mutations

The Pyrosequencing® technology was used to verify point mutations by determining the nucleotide sequence of a short, defined section of DNA. A PCR reaction was performed first to amplify a short DNA fragment containing the section to be sequenced. The PCR-amplified template needs to be single-stranded and covalently attached to a biotin molecule at its 5' end. Biotin served to attach the template non-covalently to streptavidin which was attached to a stationary phase of cross-linked agarose (sepharose).

Amplification of biotinylated DNA fragments: The PCR reaction was carried out in 96 well PCR plates. The reaction mixture contains 1 µL of forward primer solution (kerfi016 for *AtHPPD*, kerfi020 for *PfHPPD*; 10 pmol*µL⁻¹), 1 µL of reverse primer solution (contain a biotin modification at their 5' ends; kerfi019 for *AtHPPD,* kerfi014 for *PfHPPD*; 10 pmol*µL⁻¹), 2 µL of liquid bacterial culture of a clone cultivated in a deepwell plate, 25 µL of HotStarTaq® Master Mix and 21 µL of HyPure™ Molecular Biology Grade Water.

The PCR programmes for *At*HPPD and *Pf*HPPD differed concerning the annealing temperatures which were set to 55 °C and 60 °C, respectively.
1. 95 °C 15 min
2. 94 °C 30 s
   55 °C / 60 °C 30 s
   72 °C 30 s
   Step 2 was repeated 32 times.
3. 72 °C 10 min

| Primer name | Primer sequence |
|---|---|
| kerfi014 (5'bio) | 5'-GATCTTCTCGGAAACCCTGATG-3' |
| kerfi016 | 5'-GGGATTCTTGTAGACAGAGATG-3' |
| kerfi019 (5'bio) | 5'- CCCACTAACTGTTTGGCTTC-3' |
| kerfi020 | 5'- GGCGGTCAATACACCACGAC-3' |

Pyrosequencing® reaction: the Pyrosequencing® reaction (Biotage) was carried out in 96 well plates. To each 45 µL PCR reaction, 40 µL of Binding Buffer (10 mM Tris-HCl; 2 M NaCl; 1 mM EDTA; 0.1 % Tween 20), 3 µL streptavidin sepharose beads (composition proprietary - GE Healthcare BioScience AB) and 12 µL ddH₂O were added. These mixtures were shaken for 10 minutes in the 96 well PCR plate.

With a "vacuum prep tool" each solution was then drawn through a small filter attached to a small metal tube, while the streptavidin beads, now bound to the biotinylated PCR product, were retained on the filters by the suction. According to this principle, the filters were then immersed in 70% ethanol for 5 seconds to wash the DNA and remove primers, dNTPs and other components of the PCR reaction. The procedure was repeated with 0.2 M NaOH to denature dsDNA and to leave only the biotinylated DNA strand bound to the streptavidin beads. After a final washing of the DNA in Washing Buffer, the "vacuum prep tool" was held over a PSQ™ 96 plate that contained 40 µL of Annealing Buffer and 0.1 µL of Pyrosequencing® primer solution (100 pmol*µL*; kerfi018 for *AtHPPD* / kerfi015 for *PfHPPD*) per well. The vacuum was then shut off and each filter was dipped into its corresponding well to dissolve the DNA that was retained by the filter. The plate was then incubated at 80 °C for 2 min to resolve secondary structures eventually formed within the DNA templates. While the solutions cooled to room temperature the Pyrosequencing® primers hybridized to their binding sites on the template.

The remaining components of the Pyrosequencing® reactions (620 µL of enzyme mixture, 620 µL of substrate mixture and 130 µL of each dNTP solution) were filled into separate wells of a cartridge. The cartridge and the PSQ™ plate were then placed inside the PyroMark™ ID.
The Pyrosequencing® instrument automatically added enzyme and substrate to the reaction mixture before the sequencing reaction is started by addition of the first dNTP. To determine the DNA sequence downstream of the primer, a SQA-run is conducted. The order of nucleotides added to the reaction mixture is defined in advance. The PyroMark™ ID software can be used to translate the Pyrogram® traces into the DNA sequence.

### Results:

The PCR-amplified fragment of *AtHPPD* has a size of 239 bp and the biotin is attached to the non-coding strand; the *PfHPPD* fragment comprises 142 bp and the biotin is attached to the coding strand.

The mutated codon in *AtHPPD* is located three bases downstream of the kerfi018 primer sequence. The first three bases sequenced are adenines, followed by the mutated codon. The coding strand of the *AtHPPD* fragment is synthesized by the DNA polymerase, so the sequence could be directly translated into the amino acid sequence.

Screening of 438 *AtHPPD* colonies issued 146 mutant genes, 181 wild type genes (codon GGC at position 422) and 111 failed sequencing reactions or ambiguous results.

The production of mutant clones by transformation of mutant plasmids in either E. *coli* K-12 MG1655 or E. *coli* TOP10 was therefore successful in 33 % of all cases. Codons encoding all amino acids except lysine could be obtained. The genes containing the codons for glutamic acid, histidine, isoleucine, threonine, tryptophan and tyrosine were present in *E*. *coli* TOP10 clones from which DNA was prepared and transformed into *E. coli* K-12 MG1655 cells. If possible, synonymous codons were selected considering codon usage in *E*. *coli* K-12. No codon used at a frequency lower than 10 % was chosen, most selected codons are used at a frequency higher than 35 % (Codon usage database; E. coli K-12: http://www.kazusa.or.jp/codon/cgi-bin/showcodon.cgi?species=83333).

Starting from the primer kerfi015, the non-coding strand of the *PfHPPD* fragment is synthesized by the DNA polymerase, so the nucleotide sequence needed to be translated into the reverse complement before it could be translated into the amino acid sequence. The mutated codon immediately succeeds the primer and is therefore represented by the first three bases sequenced in the reaction.

Screening of 252 *PfHPPD* colonies issued 119 mutant genes, 73 unaltered genes (codon TGG at position 336) and 60 failed sequencing reactions or ambiguous results.
The production of mutant clones by transformation of mutant plasmids in *E*. *coli* K-12 MG1655 cells was therefore successful in 47 % of all cases. Codons encoding all amino acids except alanine could be obtained. If possible, synonymous codons were selected considering codon usage in *E*. *coli* K-12 as described above for *AtHPPD* codons.

| Primer name | Primer sequence |
|---|---|
| kerfi015 | 5'-GACTCGAACAGCGCCTTGAAGTT-3' |
| kerfi018 | 5'-GGATGTGGTGGTTTTGGC-3' |

### Example 3 : Assay for HPPD activity

HPPD produces homogentisate and CO₂ from 4-HPP and O₂. The enzyme is incubated with its substrate 4-HPP in the presence or absence of an inhibitor. L-ascorbic acid is present as a reductant to retain the active site iron in the ferrous form and Catalase is present to degrade toxic H₂O₂. After an incubation time of one hour, the reaction is stopped by addition of 2,4-Dinitrophenylhydrazine (DNP). DNP forms a hydrazone derivative with the remaining 4-HPP molecules in the assay mixture which appears in an amber-brown colour at an alkaline pH. The amount of unconsumed 4-HPP is measured photometrically at 405 nm.

For preparation of inhibitor stock solutions, Tembotrione (M_{w} = 440.82) and DKN (M_{w} = 359.3) are dissolved in DMSO to a concentration of 10 mM. This stock solution is first diluted 20-fold in 25 % DMSO to a concentration of 0.5 mM. Further dilutions are made with ddH₂O to obtain the inhibitor solutions used in the assay (5 µM, 10 µM and 20 µM). The respective inhibitor solution accounts for half of the assay mixture volume, meaning that its active concentration is again reduced 2-fold. This results in inhibitor concentrations of 2.5 µM, 5 µM and 10 µM. A 2 % DMSO solution provides for half of the assay mixture in uninhibited reactions to normalize a possible inhibiting effect of DMSO.

The assay is designed for a HPPD concentration of 444 nM on a monomeric basis and a 4-HPP concentration of 500 µM. This corresponds to 44.4 pmol HPPD and 50 nmol 4-HPP in a 100 µL-assay mixture, resulting in an approximate 1000-fold excess of substrate in relation to the enzyme. The calculated theoretical molecular weight of an AtHPPD subunit is 49.515 kD which results in 2.2 µg HPPD per assay mixture. The calculated theoretical molecular weight of a PfHPPD subunit is 41.205 kD, resulting in 1.8 µg HPPD per assay mixture. The enzyme solution provides for one quarter of the assay mixture volume, so enzyme stock solutions are produced by diluting *At*HPPD solutions to 88 µg*mL⁻¹ with 50 mM TRIS buffer; *Pf*HPPD solutions are diluted to 72 µg*mL⁻¹.

The inhibitor concentrations (2.5 µM, 5 µM and 10 µM) provide for 5-fold, 10-fold and 20-fold excess of inhibitor compared to the amount of enzyme. A buffer/substrate solution is prepared which provides for one quarter of the assay mixture. 2.5 mL of buffer/substrate solution contain 1 mL 1 M TRIS buffer, 500 µL 10 mM 4-HPP solution, 500 µL 200 mM L-ascorbic acid solution, 13 µL Catalase solution and 487 µL ddH₂O. The assay is carried out in Greiner F-bottom 96 well microplates and all reactions are carried out as triplicates. The controls are carried out sixfold per plate and contain either 25 µL 50 mM TRIS instead of HPPD solution (corresponding to 0 % consumption of 4-HPP) or a buffer/substrate solution that contains 500 µL 1 M TRIS instead of 500 µL 10 mM 4-HPP (corresponding to 100 % consumption of HPP). The reaction is started by addition of 25 µL HPPD solution to a mixture of 50 µL of the respective inhibitor solution or 50 µL 2% DMSO and 25 µL buffer/substrate solution. The reaction is allowed to proceed for 1 h at room temperature. The reaction is stopped and coloration of 4-HPP is induced by addition of 50 µL 0.04 % DNP/3.8 N HCl solution. After 15 min, addition of 100 µL 5 N KOH leads to the colour shift of the hydrazone derivative. Photometric measurement with a BMG FLUOstar Galaxy microplate reader is carried out immediately at 405 nm and data obtained is used for analysis of HPPD activities in presence and absence of an inhibitor.

### Results:

The *At*HPPD mutants in position 422 with reference to the amino acid sequence of the Aradiposis HPPD of SEQ ID NO4 (i.e. Gly422Ala, -Arg, -Asn, -Asp, -Cys, - Glu, -His, -Leu, -Met, -Phe, -Pro, -Ser, -Tyr, and - Val) were tested along with the WT enzyme in the assay for HPPD activity (it is noted that Gly422 with reference to the amino acid sequence of the Aradiposis HPPD of SEQ ID NO4 corresponds to Gly336 with respect to the Pseudomonas reference sequence of SEQ ID NO: 2). All enzymes were active, but only the activities of the mutants Gly422Ala, -Asn, -Asp, -Cys, -His, -Met, -Phe, -Tyr and -Val were within or above the range (≥ 70 %) of the WT enzyme. The WT enzyme retained 35 % of its activity in the presence of 2.5 µM Tembotrione; only the mutants Gly422Asn, -Cys, -His and -Val retained higher activities ranging at 39, 44, 51 and 43 %, respectively. Activities were further reduced at higher concentrations of Tembotrione. Only the mutant Gly422His displayed a residual activity of about 40 % in the presence of 5 and 10 µM Tembotrione while all other enzymes displayed activities comparable to the WT enzyme at these inhibitor concentrations, ranging at approximately 20 and 10 %, respectively (Table 1).

The PfHPPD mutants Gly336Arg, -Asp, -Gln, -Glu, - His, -Leu, -Lys, -Met, -Phe, Thr, Trp and -Pro were tested along with the WT enzyme. With exception of the Gly336Pro mutant, whose uninhibited activity ranged below 70 % of WT activity, the activities of the Gly336 mutants were within or above the range of the WT enzyme (≥ 75 %). The WT enzyme retained only 5 % of its activity in the presence of 2.5 µM Tembotrione while the mutants Gly336Asp, - Arg, -Gln, -Glu, -His, -Met, - Phe and -Trp retained activities above 14 %. The highest residual activities were those of Gly336His (26 %) and Gly336Phe (33 %). Interestingly, the Gly336His mutant displayed residual activities of 13 and 11.2 % in the presence of 5 and 10 µM Tembotrione, respectively, while the activities of Gly336Phe was reduced to 12.4 and 2.5 %, respectively. The Gly336Met mutant, displayed residual activities of 7 and 10 % respectively at these inhibitor concentrations, while the activity of the WT enzyme was reduced to zero. (Table 1).

**Table 1 : Relative activity (in percentage) of Pf HPPD and At HPPD mutants in presence and absence of Tembotrione; Activities are normalized by setting the uninhibited enzyme activity to 100%**

| **Pseudomonas fluorescens HPPD** | | | | |
|---|---|---|---|---|
| | Concentration of Tembotrione (µM) | | | |
| Gly336 mutant | 0 | 2,5 | 5 | 10 |
| Arg | 100 | 14 | 7 | 2 |
| Asp | 100 | 18 | 9 | 0 |
| Gln | 100 | 14 | 0 | 0 |
| Glu | 100 | 15 | 7 | 0 |
| Gly | 100 | 5 | 0 | 0 |
| His | 100 | 26 | 13 | 11 |
| Leu | 100 | 4 | 0 | 0 |
| Lys | 100 | 6 | 0 | 0 |
| Met | 100 | 16 | 7 | 10 |
| Phe | 100 | 33 | 12 | 3 |
| Pro | 100 | 5 | 4 | 0 |
| Thr | 100 | 8 | 2 | 2 |
| Trp | 100 | 21 | 7 | 0 |

| **Arabidopsis thaliana HPPD** | | | | |
|---|---|---|---|---|
| | Concentration of Tembotrione (µM) | | | |
| Gly422 mutant* | 0 | 2,5 | 5 | 10 |
| Ala | 100 | 25 | 21 | 15 |
| Arg | 100 | 17 | 1 | 1 |
| Asn | 100 | 39 | 26 | 15 |
| Asp | 100 | 20 | 7 | 10 |
| Cys | 100 | 44 | 27 | 19 |
| Glu | 100 | 24 | 24 | 0 |
| Gly | 100 | 35 | 21 | 12 |
| His | 100 | 50 | 31 | 40 |
| Leu | 100 | 31 | 23 | 14 |
| Met | 100 | 18 | 13 | 12 |
| Phe | 100 | 30 | 16 | 11 |
| Pro | 100 | 0 | 0 | 0 |
| Ser | 100 | 18 | 4 | 0 |
| Tyr | 100 | 26 | 11 | 0 |
| Val | 100 | 43 | 22 | 14 |

| | | | | |
|---|---|---|---|---|
| * Mutation at the gly in position 422 with reference to the amino acid sequence of the Aradiposis HPPD of SEQ ID NO4 (corresponds to Gly336 with reference to the amino acid sequence of the Pseudomonas HPPD of SEQ ID NO2) | | | | |

### Example 4: Assay for PDH activity

The prephenate dehydrogenase activity was measured at 25°C by spectrophotometric monitoring at 340 nm of the formation of NADH or NADPH in a solution containing 50 mM of tris-HCl, pH 8.6, 300 µM of prephenate, and 1 mM of NAD or NADP in a total volume of 200 µl.

### Example 3: construction of chimeric genes for the evaluation of unmutated and mutated Pf HPPD in tobacco.

### A) Construction of the chimeric genes:

The vector which is employed in order to make the constructs which HPPD (wild-type or mutants) to be expressed in type PBD6 tobacco plants is designated pRP-RD224. This vector was initially conceived for cloning all the *Pseudomonas* HPPD mutants by simply replacing the truncated HPPD gene of this vector between the KpnI and BstEII sites. Its construction from the binary vector pBI121 (Clontech) is extensively described in WO 99/24585.
Clone pRP-RD224 therefore has the following structure:
RB/Nos promoter/NPTII/Nos terminator/double histone promoter/tev/otp/truncated HPPD/Nos terminator/LB
wherein "truncated HPPD" refers to the sequence encoding the Pf HPPD truncated of approximately 500 base pairs in order subsequently to facilitate screening of the transformed colonies which have integrated the mutant HPPDs (WO99/24585)

pRP-RD224 mutants : The DNAs of the vectors carrying the mutated and unmutated HPPDs were digested with KpnI and BstEII, purified and then ligated into vector pRP-RD224, which had been digested with KpnI and BstEII and purified. The transformants which had integrated the mutated HPPD gene were selected for the size of the insert by digesting with KpnI and BstEII. The resulting clones are designated pRP-RD224 to which is added the type of mutation which has been carried out on the HPPD; in this way, the following clones were created: pRP RD224 Pf (for the unmutated enzyme), pRP RD224 PfH336 (for the enzyme having a histidine at position 336), pRP RD224 PfM336 (for the enzyme having a methionine at position 336), and pRP RD224 PfF336 (for the enzyme having a phenylalanine at position 336) .

### Example 4: Construction of a chimeric gene overexpressing PDH

The construction of a chimeric gene overexpressing PDH comprises assembling, in the direction of transcription, a "double histone" promoter (PdH4) as described in patent application EP 0 507 698, the tobacco etch virus translational enhancer (TEV) sequence described in Carrington and Freed (1990), a sequence encoding an optimized transit peptide (OTP) as described in patent application EP 0 508 909, the coding portion of the yeast PDH gene described in Mannhaupt et al. (1989) and the nos terminator of the nopaline synthase gene described in Bevan et al. (1983). The assembly was then cloned into the binary vector pRD 224 containing a kanamycin tolerance gene(NPTII), to give the vector pRD 224-PDH.

This binary vector was then used to transform the *Agrobacterium* strain EHA 105 and to give the *Agrobacterium* strain EHA 105-pRD 224-PDH. This *Agrobacterium* strain was used to transform tobacco plants transformed with the chimeric genes as described in example 3.

The transformed plants are selected on kanamycin.

### Cited references:

Abou-Zeid et al., 1995, Applied Env Microb 41: 1298-1302
Ausubel F.M. et al., "Current Protocols in Molecular Biology" Volumes 1 and 2, published by Greene Publishing Associates and Wiley Interscience (1989) Bevan et al., 1983, Nucleic Acids Res. 11(2), 369-385 Bonner et al., 1995, Plant Cells Physiol. 36, 1013-1022 Byng et al., 1981, Phytochemistry 6: 1289-1292 Carrington and Freed, 1990; J. Virol. 64: 1590-1597 Christou et al., 1991, Biotechnology 9:957 Connely and Conn, 1986, Z. Naturforsch 41c: 69-78 Crouch N.P. et al., 1997, Tetrahedron, 53, 20, 6993-7010
Datla, R. et al., 1997, Biotechnology Ann. Rev. 3, 269-296
Gaines et al., 1982, Plants 156: 233-240 Henner et al., 1986, Gene 49 (1) 147-152 Hiei et al., 1994, Plant J 6:271-282 Hiei et al., 1997, Plant Mol Biol. 35:205-21 Hudson et al., 1984, J. Mol. Biol. 180(4), 1023-1051 Fritze et al., 2004, Plant Physiology 134:1388-1400 Garcia et al., 1997, Biochem. J. 325, 761-769 Garcia et al., 1999, Plant Physiol. 119, 1507-1516 Gould J.H. and Magallanes-Cedeno M.,1998, Plant Molecular Biology reporter, 16:1-10
Horsch et al., 1985, Science 227: 1229-1231 Lingens et al., 1967, European J. Biochem 1: 363-374 Maniatis T., Fritsch E.F., in Molecular cloning, Sambrook, 1982.
Mannhaupt et al., 1989, Gene 85, 303-311 Matringe et al., 2005, Pest Management Science 61:269-276
Mitchell et al., 2001, Pest Management Science 57:120-128
Pallett et al., 2001, Pest Management Science 57:133-142
Sambrook et al., 1989, Molecular cloning: a laboratory manual, second edition, Cold Spring Harbor Laboratory Press, Cold Spring Harbor, New York) Sambrook J. and Russell D., 2001, Molecular Cloning: A laboratory Manual (third edition), ISBN 978-087969577-4 CSHL Press Sampathkumar and Morrisson, 1982, Bioch Biophys Acta 701: 204-211 Sawahel W.A.,2001, Plant Molecular Biology reporter, 19: 377a-377f
Schulz et al., 1993, FEBS Letters 318:162-166 Xia et al., 1992, J. Gen. Microbiol. 138(7), 1309-1316 Zapata C.,1999, theoretical Applied Genetics, 98 (2) :1432-2242

## Claims

1. Mutated hydroxyphenylpyruvate dioxygenase (HPPD) which retains its properties of catalysing the conversion of para-hydroxyphenylpyruvate (HPP) to homogentisate and which is less sensitive to tembotrione than the original unmutated HPPD, **characterized in that** it contains a mutation on the amino acid glycine in position 336 with reference to the amino acid sequence of the *Pseudomonas* HPPD of SEQ ID NO:2 which is Gly336Phe.

2. Mutated HPPD according to claim 1, **characterized in that** the mutated HPPD contains a second mutation.

3. Mutated HPPD according to claim 2, **characterized in that** the second mutated amino acid is selected from the selected amino acids: Pro215, Gly298, Gly332, Phe333, Gly334 and Asn337, with reference to the *Pseudomonas* HPPD sequence of SEQ ID NO:2.

4. Nucleic acid which encodes a mutated HPPD according to one of claims 1 to 3.

5. Chimeric gene which comprises a coding sequence as well as heterologous regulatory element in the 5' and optionally in the 3' positions, which are able to function in a host organism, **characterized in that** the coding sequence contains at least a nucleic acid sequence according to claim 4.

6. Chimeric gene according to claim 5 **characterized in that** it contains in the 5' position of the nucleic acid sequence which encodes a mutated HPPD, a nucleic acid sequence which encodes a plant transit peptide, with this sequence being arranged between the promoter region and the sequence encoding the mutated HPPD so as to permit expression of a transit peptide/mutated HPPD fusion protein.

7. Transit peptide/mutated HPPD fusion protein, with the mutated HPPD being defined according to one of claim 1 to 3.

8. Cloning and/or expression vector for transforming a host organism, **characterized in that** it contains at least one chimeric gene according to one of claims 5 to 6.

9. Plant cell, **characterized in that** it contains at least a nucleic acid according to claim 4 or a chimeric gene according to one of claims 5 to 6.

10. Plant cell according to claim 9 **characterized in that** it contains, in addition, a gene that is functional in plants allowing overexpression of a PDH (prephenate dehydrogenase) enzyme.

11. Transformed plant, **characterized in that** it contains a transformed plant cell according to claim 9 or claim 10.

12. Transformed seed, **characterized in that** it contains a transformed plant cell according to claim 9 or to claim 10 and **in that** it originates from a transformed plant according to claim 11.

13. Method for obtaining a plant resistant to a HPDD inhibitor, **characterized in that** the plant is transformed with a chimeric gene according to one of claims 5 to 6.

14. Method for obtaining a plant resistant to a HPDD inhibitor according to claim 13, **characterized in that** the plant is further transformed, simultaneously or successively, with a second gene functional in this plant allowing overexpression of a PDH (prephenate dehydrogenase) enzyme.

15. Method for controlling weeds in an area or a field which contains transformed seeds according to claim 12 or plants according to claim 11, which method comprises applying, to the said area of the field, a dose of a HPPD inhibitor herbicide which is toxic for the said weeds, without significantly affecting the seeds or plants which contains a nucleic acid sequence according to claim 4 or a chimeric gene according to one of claims 5 to 6.

16. Method for obtaining oil or meal comprising growing a transformed plant according to claim 11, optionally treating such plant with an HPPD inhibitor herbicide, harvesting the grains and milling the grains to make meal and optionally extract the oil.

17. The method according to any one of claims 13 to 16, in which the HPPD inhibitor is a triketone HPPD inhibitor.

18. The method according to claim 17, in which the HPPD inhibitor is selected from tembotrione, mesotrione, and sulcotrione, particularly tembotrione.

19. Use of a HPPD of claim 1 which has been mutated in the amino acid glycine at the position 336 with reference to the amino acid sequence of the *Pseudomonas* HPPD of SEQ ID NO:2 to render plants tolerant to a triketone HPPD inhibitor, **characterized in that** the HPPD mutation is Gly336Phe.

20. Use of a mutated HPPD according to claim 19, **characterized in that** the HPPD inhibitor is selected from tembotrione, mesotrione, and sulcotrione.

## Patentansprüche

1. Mutierte Hydroxyphenylpyruvatdioxygenase (HPPD), die ihre Eigenschaften des Katalysierens der Umwandlung von Parahydroxyphenylpyruvat (HPP) in Homogentisat behält und die gegenüber Tembotrion weniger empfindlich ist als die ursprüngliche unmutierte HPPD, **dadurch gekennzeichnet, dass** sie eine Mutation an der Aminosäure Glycin in Position 336 unter Bezugnahme auf die Aminosäuresequenz der Pseudomonas-HPPD von SEQ ID NO:2 enthält, die Gly336Phe ist.

2. Mutierte HPPD nach Anspruch 1, **dadurch gekennzeichnet, dass** die mutierte HPPD eine zweite Mutation enthält.

3. Mutierte HPPD nach Anspruch 2, **dadurch gekennzeichnet, dass** die zweite mutierte Aminosäure ausgewählt ist aus den ausgewählten Aminosäuren: Pro215, Gly298, Gly332, Phe333, Gly334 und Asn337 unter Bezugnahme auf die Pseudomonas-HPPD-Sequenz von SEQ ID NO:2.

4. Nukleinsäure, die eine mutierte HPPD nach einem der Ansprüche 1 bis 3 codiert.

5. Chimäres Gen, das eine Codiersequenz sowie ein heterologes regulatorisches Element in den 5'- und optional in den 3'-Positionen umfasst, die fähig sind, in einem Wirtsorganismus zu arbeiten, **dadurch gekennzeichnet, dass** die Codiersequenz mindestens eine Nukleinsäuresequenz nach Anspruch 4 enthält.

6. Chimäres Gen nach Anspruch 5, **dadurch gekennzeichnet, dass** es in der 5'-Position der Nukleinsäuresequenz, die eine mutierte HPPD codiert, eine Nukleinsäuresequenz enthält, die ein Pflanzentransitpeptid codiert, wobei diese Sequenz zwischen der Promotorregion und der Sequenz angeordnet ist, welche die mutierte HPPD codiert, um die Expression eines Transitpeptids/mutierten HPPD-Fusionsproteins zu erlauben.

7. Transitpeptid/mutiertes HPPD-Fusionsprotein, wobei die mutierte HPPD nach einem von Anspruch 1 bis 3 definiert ist.

8. Klonierungs- und/oder Expressionsvektor zum Transformieren eines Wirtsorganismus, **dadurch gekennzeichnet, dass** er mindestens ein chimäres Gen nach einem der Ansprüche 5 bis 6 enthält.

9. Pflanzenzelle, **dadurch gekennzeichnet, dass** sie mindestens eine Nukleinsäure nach Anspruch 4 oder ein chimäres Gen nach einem der Ansprüche 5 bis 6 enthält.

10. Pflanzenzelle nach Anspruch 9, **dadurch gekennzeichnet, dass** sie außerdem ein Gen enthält, das in Pflanzen funktionsfähig ist, die eine Überexpression eines PDH-(Prephenatdehydrogenase) -Enzyms ermöglichen.

11. Transformierte Pflanze, **dadurch gekennzeichnet, dass** sie eine transformierte Pflanzenzelle nach Anspruch 9 oder Anspruch 10 enthält.

12. Transformierter Samen, **dadurch gekennzeichnet, dass** er eine transformierte Pflanzenzelle nach Anspruch 9 oder Anspruch 10 enthält und dass er von einer transformierten Pflanze nach Anspruch 11 herstammt.

13. Verfahren zum Erlangen einer Pflanze, die gegenüber einem HPDD-Inhibitor resistent ist, **dadurch gekennzeichnet, dass** die Pflanze mit einem chimären Gen nach einem der Ansprüche 5 bis 6 transformiert ist.

14. Verfahren zum Erlangen einer Pflanze, die gegenüber einem HPDD-Inhibitor nach Anspruch 13 resistent ist, **dadurch gekennzeichnet, dass** die Pflanze ferner gleichzeitig oder nacheinander mit einem zweiten Gen transformiert wird, das in dieser Pflanze funktionsfähig ist, was eine Überexpression der Pflanze eines PDH-(Prephenatdehydrogenase) -Enzyms ermöglicht.

15. Verfahren zum Bekämpfen von Unkraut in einem Bereich oder einem Feld, das transformierte Samen nach Anspruch 12 oder Pflanzen nach Anspruch 11 enthält, wobei das Verfahren das Anwenden einer Dosis eines HPPD-Inhibitorherbizids, das für das Unkraut giftig ist, auf den Bereich des Feldes umfasst, ohne die Samen oder Pflanzen erheblich zu beeinflussen, das eine Nukleinsäuresequenz nach Anspruch 4 oder ein chimäres Gen nach einem der Ansprüche 5 bis 6 enthält.

16. Verfahren zum Erlangen von Öl oder Mehl, umfassend das Züchten einer transformierten Pflanze nach Anspruch 11, optional das Behandeln einer solchen Pflanze mit einem HPPD-Inhibitorherbizid, das Ernten der Körner und Mahlen der Körner, um Mehl herzustellen und optional das Öl zu extrahieren.

17. Verfahren nach einem der Ansprüche 13 bis 16, wobei der HPPD-Inhibitor ein Triketon-HPPD-Inhibitor ist.

18. Verfahren nach Anspruch 17, wobei der HPPD-Inhibitor ausgewählt ist aus Tembotrion, Mesotrion und Sulcotrion, insbesondere Tembotrion.

19. Gebrauch einer HPPD nach Anspruch 1, die im Aminosäureglycin an der Position 336 unter Bezugnahme auf die Aminosäuresequenz der Pseudomonas-HPPD von SEQ ID NO:2 mutiert wurde, um Pflanzen zu machen, die gegenüber einem Triketon-HPPD-Inhibitor tolerant sind, **dadurch gekennzeichnet, dass** die HPPD-Mutation Gly336phe ist.

20. Gebrauch einer mutierten HPPD nach Anspruch 19, **dadurch gekennzeichnet, dass** der HPPD-Inhibitor ausgewählt wird aus Tembotrion, Mesotrion und Sulcotrion.

## Revendications

1. Hydroxyphénylpyruvate dioxygénase (HPPD) mutée qui conserve ses propriétés de catalyse de la conversion de para-hydroxyphénylpyruvate (HPP) en homogentisate et qui est moins sensible à la tembotrione que la HPPD non mutée d'origine, **caractérisée en ce qu'**elle contient une mutation sur l'acide aminé glycine en position 336 en référence à la séquence d'acides aminés de la HPPD de *Pseudomonas* de SEQ ID NO : 2 qui est Gly336Phe.

2. HPPD mutée selon la revendication 1, **caractérisée en ce que** la HPPD mutée contient une deuxième mutation.

3. HPPD mutée selon la revendication 2, **caractérisée en ce que** le deuxième acide aminé muté est choisi par les acides aminés choisis : Pro215, Gly298, Gly332, Phe333, Gly334 et Asn337, en référence à la séquence de HPPD de *Pseudomonas* de SEQ ID NO : 2.

4. Acide nucléique qui code pour une HPPD mutée selon l'une des revendications 1 à 3.

5. Gène chimère qui comprend une séquence codante ainsi qu'un élément régulateur hétérologue en position 5' et éventuellement en position 3', qui sont capables de fonctionner dans un organisme hôte, **caractérisé en ce que** la séquence codante contient au moins une séquence d'acides nucléiques selon la revendication 4.

6. Gène chimère selon la revendication 5, **caractérisé en ce qu'**il contient en position 5' de la séquence d'acides nucléiques qui code pour une HPPD mutée, une séquence d'acides nucléiques qui code pour un peptide de transit végétal, cette séquence étant disposée entre la région de promoteur et la séquence codant pour la HPPD mutée de façon à permettre l'expression d'un protéine de fusion peptide de transit/HPPD mutée.

7. Protéine de fusion peptide de transit/HPPD mutée, la HPPD mutée étant définie selon l'une des revendications 1 à 3.

8. Vecteur de clonage et/ou d'expression destiné à transformer un organisme hôte, **caractérisé en ce qu'**il contient au moins un gène chimère selon l'une des revendications 5 à 6.

9. Cellule végétale, **caractérisée en ce qu'**elle contient au moins un acide nucléique selon la revendication 4 ou un gène chimère selon l'une des revendications 5 à 6.

10. Cellule végétale selon la revendication 9, **caractérisée en ce qu'**elle contient également un gène qui est fonctionnel dans les végétaux, permettant une surexpression d'enzyme PDH (préphénate désyhdrogénase).

11. Végétal transformé, **caractérisé en ce qu'**il contient une cellule végétale transformée selon la revendication 9 ou 10.

12. Semence transformée, **caractérisée en ce qu'**elle contient une cellule végétale transformée selon la revendication 9 ou la revendication 10, et **en ce qu'**elle provient d'un végétal transformé selon la revendication 11.

13. Procédé d'obtention d'un végétal résistant à un inhibiteur de HPPD, **caractérisé en ce que** le végétal est transformé avec un gène chimère selon l'une des revendications 5 à 6.

14. Procédé d'obtention d'un végétal résistant à un inhibiteur de HPPD selon la revendication 13, **caractérisé en ce que** le végétal est aussi transformé, simultanément ou successivement, avec un deuxième gène fonctionnel dans le végétal, permettant une surexpression d'une enzyme PDH (préphénate déshydrogénase).

15. Procédé de lutte contre la mauvaise herbe dans une zone ou un champ qui contient des semences transformées selon la revendication 12 ou des végétaux selon la revendication 11, le procédé comprenant l'application, à ladite zone ou audit champ, d'une dose d'herbicide inhibiteur de HPPD qui est toxique pour lesdites mauvaises herbe, sans affecter de manière significative les semences ou les végétaux qui contiennent une séquence d'acides nucléiques selon la revendication 4 ou un gène chimère selon l'une des revendications 5 à 6.

16. Procédé d'obtention d'huile ou de farine comprenant la culture d'un végétal transformé selon la revendication 11, éventuellement le traitement de ce végétal avec un herbicide inhibiteur de HPPD, la récolte des graines et la mouture des grains pour produire de la farine et éventuellement extraire l'huile.

17. Procédé selon l'une quelconque des revendications 13 à 16, dans lequel l'inhibiteur de HPPD est un inhibiteur de HPPD à base tricétone.

18. Procédé selon la revendication 17, dans lequel l'inhibiteur de HPPD est choisi parmi la tembotrione, la mésotrione et la sulcotrione, en particulier la tembotrione.

19. Utilisation d'une HPPD de la revendication 1 qui a été mutée dans l'acide aminé glycine en position 336 en référence à la séquence d'acides aminés de HPPD de *Pseudomonas* de SEQ ID NO : 2 pour rendre des végétaux tolérants à un inhibiteur de HPPD à base de tricétone, **caractérisée en ce que** la mutation de HPPD est Gly336Phe.

20. Utilisation d'une HPPD mutée selon la revendication 19, **caractérisée en ce que** l'inhibiteur de HPPD est choisi parmi la tembotrione, la mésotrione et la sulcotrione.
